# EUROPEAN PATENT SPECIFICATION

(11) **EP 2 117 590 B1**
(45) Date of publication and mention of the grant of the patent: **22.06.2011**
(21) Application number: 07857167.6
(22) Date of filing: 31.12.2007
(51) Int. Cl.: A61K 39/145

(54) **NON-SPECIFIC IMMUNOSTIMULATING AGENTS**
NICHT-SPEZIFISCHE IMMUNSTIMULIERENDE MITTEL
AGENTS IMMUNOSTIMULANTS NON SPECIFIQUES

(30) Priority: 29.12.2006 EP 06027120
(43) Date of publication of application: 18.11.2009
(73) Proprietor: Pevion Biotech Ltd., 3063 Ittigen (CH)
(72) Inventor: MOSER, Christian, CH-3122 Kehrsatz (CH); KAMMER, Andreas, CH-3052 Zollikofen (CH); ZURBRIGGEN, Rinaldo, CH-3185 Schmitten (CH)
(74) Representative: Grünecker, Kinkeldey, Stockmair & Schwanhäusser Anwaltssozietät
(86) International application number: PCT/EP2007/011477
(87) International publication number: WO 2008/080628

(56) References cited:
- EP-A- 1 447 080
- WO-A-2006/100110
- US-A1- 2006 275 777
- HUCKRIEDE A ET AL: "The virosome concept for influenza vaccines" VACCINE, BUTTERWORTH SCIENTIFIC. GUILDFORD, GB, vol. 23, 8 July 2005 (2005-07-08), pages S26-S38, XP004986025 ISSN: 0264-410X
- SCHUMACHER ET AL: "Efficient induction of tumoricidal cytotoxic T lymphocytes by HLA-A0201 restricted, melanoma associated, L27Melan-A/MART-126-35 peptide encapsulated into virosomes in vitro" VACCINE, BUTTERWORTH SCIENTIFIC. GUILDFORD, GB, vol. 23, no. 48-49, 1 December 2005 (2005-12-01), pages 5572-5582, XP005171549 ISSN: 0264-410X
- CUSI MARIA GRAZIA ET AL: "Immune-reconstituted influenza virosome containing CD40L gene enhances the immunological and protective activity of a carcinoembryonic antigen anticancer vaccine." JOURNAL OF IMMUNOLOGY (BALTIMORE, MD. : 1950) 1 JUN 2005, vol. 174, no. 11, 1 June 2005 (2005-06-01), pages 7210-7216, XP002431504 ISSN: 0022-1767
- MASTROBATTISTA E ET AL: "Targeting influenza virosomes to ovarian carcinoma cells" FEBS LETTERS, ELSEVIER, AMSTERDAM, NL, vol. 509, no. 1, 30 November 2001 (2001-11-30), pages 71-76, XP004329146 ISSN: 0014-5793
- CORREALE P ET AL: "Tumour-associated antigen (TAA)-specific cytotoxic T cell (CTL) response in vitro and in a mouse model, induced by TAA-plasmids delivered by influenza virosomes" EUROPEAN JOURNAL OF CANCER, PERGAMON PRESS, OXFORD, GB, vol. 37, no. 16, November 2001 (2001-11), pages 2097-2103, XP004307931 ISSN: 0959-8049
- FELNEROVA D ET AL: "Liposomes and virosomes as delivery systems for antigens, nucleic acids and drugs" CURRENT OPINION IN BIOTECHNOLOGY, LONDON, GB, vol. 15, no. 6, December 2004 (2004-12), pages 518-529, XP004653484 ISSN: 0958-1669
- SCHUMACHER R ET AL: "Influenza virosomes enhance class I restricted CTL induction through CD4+ T cell activation" VACCINE, BUTTERWORTH SCIENTIFIC. GUILDFORD, GB, vol. 22, no. 5-6, 26 January 2004 (2004-01-26), pages 715-724, XP004485203 ISSN: 0264-410X

## Description

The present invention relates to a use of a lipid vesicle for the preparation of a medicament for non-specifically stimulating the immune response of an animal to a disease or disorder. The invention further relates to a method of non-specifically stimulating the immune response of an animal, i.e. treating, eliminating and/or preventing a disease or disorder, involving administering a lipid vesicle to an animal in need thereof. The lipid vesicle comprises, in its lipid membrane, at least one viral envelope protein.

It is desirable to increase the general resistance against diseases, especially against infectious diseases by means of non-specific stimulation of the body's immune system. At the same time the importance of the immune system for the suppression and elimination of neoplastic diseases has become evident and generally accepted. However, the mechanisms of action of general immunostimulants are unknown and subject to speculation, due to the often complex compositions and the multiple interactions with different organ systems. The increasing understanding of the interplay between innate defense mechanisms and adaptive immunity has provided plausible explanations. In particular the discovery of toll-like receptors in the 1990s, a sensor system for danger signals, has initiated a new field of research dealing with highly complex, integrated and multi-redundant defense mechanisms against invading microorganisms that provides plausible - yet incomplete - explanations for enhanced resistance against disease.

Generally, the immune system may be subdivided into two parts: the innate immune system and the adaptive immune system. As the name suggests, the innate immune system is present at birth, and provides a first defense against pathogens, yet without having the capacity to react to and neutralize any one pathogen in particular. For this reason, the innate immune system is sometimes also referred to as the unspecific immune system, and is described as using non-clonal defense mechanisms, since no individual cell clones are necessary to effect its immune response. While the innate immune system includes such structures as the acidic coating of the skin and the intact epidermis itself, it also includes more complex entities such as the complement system, antimicrobial enzyme systems as well as nonspecific mediators such as interferons and interleukins. Associated with the latter is the general inflammatory response, which also plays a role in innate immunity. At the cellular level, and partially also involved in the inflammatory response, the innate immune system includes granulocytes, the monocyte-macrophage system and the natural killer (NK) cells, the latter constituting part of the connection between nonspecific innate, and specific adaptive immune responses. One of the distinguishing features of the innate immune response is that it is comparatively rapid in its ability to combat pathogens, providing initial protection against pathogens during the initial phase in which the host's more specific adaptive immune response is being activated or developed for the first time.

The specific, or adaptive immune response requires more time to be activated or develop, and therefore follows the innate immune response. In the course of the adaptive immune response, the host combats pathogens based on past or new experience with the pathogen, or a combination of both. The adaptive immune response itself may be cellular (i.e. associated with the cytotoxic activity of specific cell clones such as cytotoxic T cells (CTCs)), or humoral (i.e. associated with antibodies produced by specific B cell clones), or a combination of both, the predominance of which arm of the adaptive immune response - cellular or humoral - being determined in part by the particular mixture of cytokines released. The migration of antigen presenting cells (APCs) from peripheral tissues into the lymphatic organs triggers the specific immune response and is also responsible for the later memory function exercised by both T and B lymphocytes, clonal populations of each of which are expanded as needed. This memory function which develops upon initial exposure to an antigen dramatically shortens the reaction time required by the adaptive immune system to mount a specific defense against the same antigen at a later time.

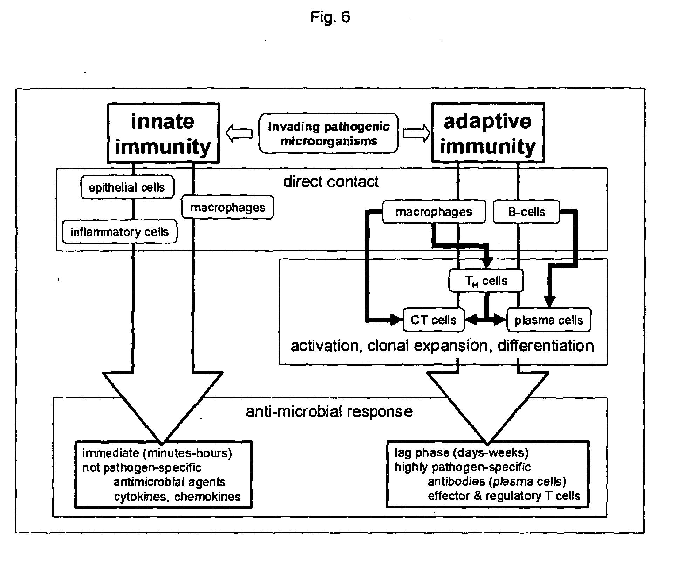

A diagram illustrating the characteristics and relationship between innate and adaptive immunity is shown in Fig. 6.

Many potential immune enhancers have been described in the literature, ranging from small synthetic molecules (poly I:C, Levamisole) to living microorganisms (Corynebacterium parvum), and including complex mixtures of bacterial components with mineral oils (Freund's adjuvant) or inorganic salts (aluminum and magnesium hydroxide/phosphate) and, more recently, recombinant proteins modulating immunity (eg cytokines, antibodies against cellular receptors). These substances have been used predominantly as adjuvants for vaccines, i.e. in combination with a specific antigen for the purpose of enhancing the immunity/resistance against the disease with which the antigenic components of the vaccine are associated. Among the large number of known immunostimulants, only very few are actually accepted adjuvants for human vaccines (Alum salts, virosomes, MF59, RC529). Most approaches have not progressed beyond pure preclinical research, leaving relevant regulatory (safety, toxicity) and economic (production costs, product profiles) aspects unaddressed. Other adjuvants are in varying stages of clinical development (e.g. bacterial toxins, Saponin derivatives, LPS and Lipid A derivatives, CpGs, nanoparticles) or are accepted for veterinary use only (e.g. ISCOMS, GERBU). The problem is to strike an acceptable balance between stimulatory effect and reactogenicity, thus the capacity to produce adverse reactions. Newly developed adjuvants bear the additional risk of unexpected side effects, as for example observed with heat labile toxin from E.coli as an adjuvant for a nasal influenza vaccine.

None of the adjuvants approved for specific vaccines is used to stimulate disease resistance in a non-specific manner, e.g. as a stand-alone product.

An increasing number of functional food or food supplement products has appeared on the market, claiming to increase disease resistance, yet often based on esoteric arguments rather than scientific evidence. These products are sold without restrictions in supermarkets, and their route of application is usually oral. Their composition can vary from well-defined vitamin or trace element cocktails to complex extracts from organic matter (plants, microorganisms, animals), and includes the so-called probiotics that contain live microorganisms (e.g. Actimel® containing L. casei defensis).

Another category of vaccine-like products such as for example Broncho-Vaxom®, Buccalin® and Uro-Vaxom® are registered medical products. These are either freely available as over the counter ("OTC") medications or as prescribed drugs, and are sold with precise indications, e.g. for the prophylaxis and treatment of infections of the respiratory and lower urinary tract: They are composed of a specific cocktail of inactivated bacteria types which are frequently associated with the respective diseases. In sharp contrast to "real" prophylactic vaccines, the treatment schedule foresees daily oral applications over an extended period of time, broth for disease prevention and treatment. The products show protective effects in preclinical models and have documented effects in humans (T cell activation, increased interferon responses and IgA levels), although the mode of action remains unclear.

Baypamun®/Zylexis® (Pfizer) is an example of an injectable immunostimulant for veterinary use. The product contains inactivated ovine parapoxvirus as active ingredient and is recommended for the prevention and treatment of infectious or stress-induced diseases in pets and farm animals. Controlled studies showed efficacy at the level of reduction of clinical symptoms in several species (cattle, horse, cat, dog, pig). The unspecific immunostimulatory effect is ascribed to the induction of cytokines, in particular of interferon. According to the product sheet, the immunostimulatory effect starts a few hours after injection and lasts up to 14 days. These kinetics are ideal for the treatment of infected herds in a situation where a specific immune prophylaxis is too late to be effective. The observed positive effects in animals have led to off-label use of Zylexis^{®} in humans, even against recommendations of health authorities, mostly in cancer patients.

Interferons are widely used as an at least partially effective antiviral treatment (viral hepatitis) that is not pathogen-specific. The recombinant proteins are given i.v. and enhance/mimic one of many effector functions of a natural unspecific response against viral infection. However, the use of interferons is restricted due to severe side effects, high costs and long treatment durations.

Levamisole (Ergamisol®), a synthetic imidazothiazole derivative, is an antibiotic drug used in combination with fluorouracil to treat colon cancer. It was originally developed and used as an antihelminthic in both humans and animals. Its mechanism of action against worms is well documented. In treating colon cancer the mechanism is documented but completely unclear. Levamisole has been shown to have immunostimulating properties. It is also used infrequently to treat melanoma and head and neck cancer.

WO 2006/085983 discloses the use of viral adjuvants for enhancing the immune response to an immunogen. The viral adjuvants described are replicating, but propagation-defective virus particles containing the viral genome in either modified or unmodified form. A disadvantage of using such viral particles as non-specific immunostimulators is that at least parts of the viral genome must be introduced into the patient with unforeseeable effects. Further, the viral particles introduced into the patient must be replication-competent.

Virosomes are semi-synthetic complexes composed of lipids and at least one viral envelope protein, produced by an in vitro procedure. The lipids are either purified from eggs or plants or produced synthetically, and a fraction of the lipids originates from the virus providing the envelope protein. Essentially, virosomes represent reconstituted, empty virus envelopes, often derived from one or more influenza viruses, devoid of the nucleocapsid including the genetic material of the source virus(es). One type of virosome is the immunopotentiating reconstituted influenza virosome ("IRIV"), which bears influenza hemagglutinin ("HA"), an influenza envelope protein which plays a key role in the fusion of influenza with target cells, embedded in its lipid membrane. Virosomes are not able to replicate but are pure fusion-active vesicles. For this reason, virosomes, like liposomes, are typically used to deliver a substance (e.g. an immunogenic molecule, a drug and/or a gene) to a target cell. But unlike liposomes, virosomes offer the advantage of efficient entry into the cells followed by the intracellular release of the virosomal contents triggered by the viral envelope protein, for example HA in the case of IRIV. Moreover, due to the incorporation of active viral envelope proteins into their membranes, virosomes release their contents into the cytoplasm immediately after being taken up by the cell, thereby preventing the degradation of the therapeutic substance in the acidic environment of the endosome (US 6040167).

In contrast to virus-like particles (VLPs), virosomes do not form spontaneously upon recombinant expression of the protein in an appropriate expression system but are the result of a controlled *in vitro* process, which allows large-scale industrial production of virosomes. The resulting virosomes contain a lipid bilayer composed mainly of synthetic lipids, whereas VLPs are made of cellular lipids and, most of the time, form no bilayers. Furthermore, in case of virosomes the content of the single components i.e. lipids, virus proteins can be varied according to the requirements for the final product.

Virosomes have been especially useful in the field of vaccination, where it is desired to stimulate an immune response to an antigen associated with a particular disease or disorder. In such cases, the antigen is typically encapsulated in or bound to the virosome, which then delivers this antigen to the host immune system to be vaccinated. By virtue of the particular antigen delivered, the resulting prophylactic and/or therapeutic effect is necessarily specific for the disease or disorder with which the antigen is associated.

Virosomes can further be loaded simultaneously with several different B-cell and T-cell epitopes (Pötl-Frank et al. (1999). Clin. Exp. Immunol. 117, 496; Moreno et al. (1993). J. Immunol. 151, 489) including universal T-helper cell epitopes (Kumar et al. (1992). J. Immunol. 148, 1499-1505) and others known to those of skill in the art. Thus, virosomes are highly effective adjuvants in modem vaccination, possessing superior properties as antigen delivery vehicles and a strong immunogenic potential while at the same time minimizing the risk of side effects.

Virosomes are functional, in that their membrane fusion activity closely mimics the well-defined low-pH-dependent membrane fusion activity of the intact virus, which is solely mediated by the viral envelope protein. Like viruses, virosomes are rapidly internalized by receptor-mediated endocytosis or opsonization. In contrast to viral systems virosomes are safe, since virosomes lack the infectious nucleocapsid of the parental virus. Thus, virosomes represent a promising carrier system for the delivery of a wide variety of different substances, either encapsulated in their aqueous interior or co-reconstituted in their membranes. Co-reconstitution of different receptors within the virosomal membrane, furthermore, allows the targeting of virosomes to different cells or tissues. Virosomes are mainly used as vaccines by adding antigen onto the surface of the virosomes or by encapsulating antigen in the virosomal lumen or into the lumen of liposomes, with which virosomes exert an adjuvant effect.

Virosomes are reconstituted from influenza virus envelopes and use the same cell receptor-mediated endocytosis as their viral counterparts (Hernandez et al. (1996). Annu Rev Cell Dev Biol 12, 627-661). The receptor binding and the membrane fusion activity of influenza virus with endosomes are known to be mediated by the major viral envelope glycoprotein HA (Bungener et al. (2002). J Liposome Res 12, 155-163; Huckriede et al. (2003). Vaccine 21, 925-931). Similar to viral vectors, the mildly acidic pH in the lumen of endosomes triggers the fusion of virosomal with endosomal membranes and thus the release of encapsulated material such as DNA, RNA, or proteins into the cytosol of APCs. Therefore, exogenous antigens encapsulated in virosomes may access the MHC class I pathway without the need of *de novo* protein synthesis. Not all virosomes are likely to fuse with endosomal membranes, and therefore a fraction is thought to become available for the MHC class II pathway.

Commercially available vaccines against influenza based on empty IRIV (INFLEXAL^{®} V) have been shown to be very efficacious and safe (Glück et al. (1994). Lancet 344, 160-163). The potential of virosomes as a delivery system for antigens associated with other specific diseases besides influenza has been demonstrated for nucleic acids and peptide-based vaccines, e.g. for malaria (Pöltl-Frank et al. (1999). Clin Exp Immunol 117, 496-503) and hepatitis A (Holzer et al. (1996) Vaccine 14, 982-986), such as with EPAXAL^{®}. Recent reports also concluded that synthetic peptide vaccines administrated s.c. (subcutaneously) with virosomes were able to induce a strong CTL immunity (Amacker et al. (2005). Int Immunol 17, 695-704).

WO 2004/045582 describes that, under appropriate *in vitro* conditions, an empty virosome (i.e. a virosome neither containing nor bearing an antigenic molecule of interest) can be made to fuse with a liposome containing or bearing such a molecule. When the resulting fusogenic particle, containing or bearing the antigenic molecule, is administered to a host, the elicited specific immune response to the antigenic molecule is greater than if this antigenic molecule were to be administered in the liposome alone. Similarly, in EP 05027624.5 it is described that a combination of empty virosomes and liposomes bearing or containing an antigenic molecule and existing as non-fused, separate entities in solution, is capable of potentiating a host immune response to a greater extent than achieved with the antigen-containing liposome alone.

Huckriede et al. (2005). Vaccine 23S1, S1/26-S1/38 reviews the use of IRIV as influenza vaccines, as well as the use of IRIV encapsulating various disease-specific antigens as vaccines against the specific diseases with which the antigens are associated. In each case, the capacity of IRIV to be used as a vaccine against a particular disease is linked to the presence of an antigen in or on the IRIV, where the antigen is associated with the specific disease to be vaccinated against.

However, as efficient as virosomes are in their direct delivery of antigenic molecules or adjuvant activity in potentiating the immune response of a host against such antigenic molecules, virosome activity as described in the prior art is specific in nature, meaning that it manifests itself in the prophylaxis, treatment and/or elimination of a specific disease or disorder dictated and limited by the nature of the antigenic molecule delivered.

In light of the above, there exists a need for further immunostimulators which do not involve introducing potentially hazardous viral genomes and replicating viral particles into patients, but which are able to mobilize the immune system in a broad, compartment-independent fashion. Such immunostimulators must be safe, based on a proven mechanism of action, and reliable in their activity.

It is an object of the invention to address this need.

It has now been surprisingly found that a virosome comprising, in its lipid membrane, at least one viral envelope protein may be used to effect a stimulation of the immune system against diseases and disorders which are not associated with the parental virus from which the at least one viral envelope protein is derived. This is surprising, as such lipid vesicles neither bear nor contain any antigenic molecules associated with any particular disease beyond the at least one viral envelope protein, or drugs. Still, the inventors have found that such empty lipid vesicles are effective in eliciting a non-specific protective effect even in the absence of any further drugs or substances associated with such diseases.

Accordingly, one aspect of the invention relates to the use of a virosome comprising, in its lipid membrane, at least one viral envelope protein, for the preparation of a medicament for non-specifically stimulating the immune response of an animal to prevent and/or effect therapy of a neoplastic, viral or bacterial disease or disorder,
wherein said neoplastic, viral or bacterial disease or disorder is not associated with or caused by the virus from which the at least one one viral envelope protein is derived;
wherein the virosome is an empty virosome which comprises no drug or substance associated with said disease or disorder, and
wherein the least one viral envelope protein is an influenza virus envelope protein.

A further aspect of the invention relates to a virosome comprising, in its lipid membrane, at least one viral envelope protein, for use in non-specifically stimulating the immune response of an animal to prevent and/or effect therapy of a neoplastic, viral or bacterial disease or disorder,
wherein said neoplastic, viral or bacterial disease or disorder is not associated with or caused by the virus from which the at least one one viral envelope protein is derived;
wherein the virosome is an empty virosome which comprises no drug or substance associated with said disease or disorder, and
wherein the least one viral envelope protein is an influenza virus envelope protein.

The virosome according to the inventive use is described in detail herein below.

Without being bound by theory, the inventors attribute the surprising effect observed to the fact that the virosomes of the invention present the host immune system with pathogen-associated molecular patterns ("PAMPs") in the form of the at least one viral envelope protein. The presence of such PAMPs alerts the immune system via the stimulation of local sensors (toll-like receptors and the like) which then lead to an activation of the innate immune system. This activation appears to be accomplished in a manner independent of any specific disease, as would normally be expected by an activation of, say, specific clonal subpopulations within the T cell and/or B cell compartments of the adaptive immune system. As a result of this global immunostimulation, the immune system is brought into a temporary state of alertness, reducing the response time to microbiological threats, increasing the magnitude of the non-specific immediate (innate) response, enhancing the subsequent development of the specific adaptive immunity and, in the event that systemic infection ensues, decreasing the severity of the symptoms associated with this infection. Essentially, then, the virosomes of the invention function as a rapid immunological "wake-up call" of sorts to increase the resistance against a broad range of diseases for a limited period of time.

As will be shown herein below in the appended examples, the inventors have demonstrated that an (empty) virosome comprising, in its lipid membrane, at least one viral envelope protein can be used to lessen the severity of a disease. Specifically, it has been shown that a virosome comprising, in its lipid membrane, at least one viral envelope protein leads to an immunostimulatory effect only hours after administration. The onset of this effect is quite rapid, and is certainly more rapid than any immunostimulatory effect arising from a specific, i.e. adaptive immune response would be expected to be. It has further been shown that, in cases where a disease was contracted by animals pretreated with the above virosome, both the severity as well as duration of the course of this disease was lessened in treated animals as compared to non-treated animal controls. Finally, while this effect was transient, it was observed for a number of different diseases, demonstrating the non-specific nature of the immunopotentiation achieved using such virosomes.

As used herein, the term "virosome" refers to a sphere bounded by a lipid bilayer and defining a lumen with a diameter on the order of 20-1000 nm, preferred 20-500 nm, more preferred 80-500 nm, even more preferred 100-200 nm. Most preferred, the diameter of the virosome is about 150 nm. "Virosome" as used herein refers to a lipid vesicle with at least one viral envelope protein in its lipid membrane, and belongs to the class of compounds termed "proteoliposomes".

As used herein, the phrase "in its lipid membrane" refers to a physical attachment of the viral envelope protein to the virosome via a transmembrane/anchor domain within the protein molecule, or via a covalently linked lipophilic molecule providing the anchor function, or via non-covalent association, either directly with components of the lipid bilayer or with molecules anchored in the lipid bilayer and, as such, is available for binding to a corresponding receptor on a cell with which the virosome may fuse.

As used herein, the term "viral envelope protein" refers to any protein encoded by an enveloped influenza virus from which the virosome used in the invention is partly or completely derived and that is present in its lipid membrane. In many cases (but not always), viral envelope proteins are part of the outer virion surface and interact with the host organisms, e.g. with receptors on the surface of cells or with soluble molecules. While a "viral envelope protein" may in some cases represent an immunogenic or antigenic molecule associated with the parental influenza virus, the viral envelope protein of the virosome used in the present invention is not incorporated with the intention of eliciting any kind of specific immune response against this protein, but rather to trigger at least one immediate, non-specific danger signal long before a specific immune response can develop. Pre-existing specific immunity against the influenza viral envelope protein explicitly does not abolish the function of the envelope protein in the sense of the present invention. Viral envelope proteins sometimes function as "viral fusion proteins", which means essentially the same thing as "viral fusion-promoting proteins", meaning that such proteins play a role in the fusion of viruses or virosomes with target cells.

As used herein, the term "immune response" refers to an increased resistance of an animal to at least one disease or disorder, including simultaneous resistance to multiple diseases or disorders. An immune response is a physiological response in humans and other higher animals to defend the body against introduction of foreign material and/or its pathological own material (*e.g.* in the context of cancer, autoimmune disease or disorder).

As used herein, the terms "non-specific", "unspecific" and the like refer to a general immunostimulatory activity of the virosome against at least one disease or disorder which is not associated with or caused by a virus from which the at least one viral envelope protein is derived. A virus from which a viral envelope protein in the lipid membrane of the virosome is derived is referred to herein as a "parental virus". "Non-specific" immunostimulation thus refers to prevention, combating and/or elimination of any one or more of many diseases or disorders not caused by the parental virus. A hallmark of the non-specific immunostimulation described herein is that its onset is very rapid following administration of the virosome. This transient non-specific immunostimulation is generally on the order of less than 5 days, but may develop even shorter after administration of the virosome, for example less than 4 days, less than 3 days, less than 2 days, less than 1 day, or even within hours following administration of the virosome.

Conversely, "specific immunostimulatory activity refers to the stimulation of the immune system to prevent, combat and/or eliminate a particular disease or disorder associated with or caused by the parental virus. The onset of this immunostimulation is slow, e.g. on the order of two weeks.

As an example, using a virosome comprising, in its lipid membrane, a viral envelope protein from parental virus A to prepare a medicament to effect immunostimulation against disease A (associated with or caused by parental virus A) would be an example of eliciting "specific" immunostimulatory activity. In contrast, using the same virosome to prepare a medicament to prevent, combat (i.e. treat) and/or eliminate disease B (not associated with or caused by parental virus A) would be an example of eliciting "non-specific, or "unspecific" immunostimulatory activity. This also applies to different strains of the same virus. For example, immunostimulation against one particular strain of a virus (e.g. influenza H1N1) effected by a virosome bearing a viral envelope protein of another particular strain (e.g. H3N2 or H5N1) belonging to the same class of virus (here, influenza) would therefore be considered non-specific immunopotentiation.

As used herein, the terms "therapeutic", "therapy" and the like refer to action taken in combating at least one disease or disorder which has already been contracted, or which is suspected of already having been contracted, regardless of whether any corresponding symptoms have already set in. As such, "therapy" and "therapeutic" refer to the treatment, elimination or at least amelioration of a disease or disorder in a subject such that, if symptoms are already present, these are mitigated or, if no symptoms are yet present, the onset of such symptoms is lessened in severity or excluded altogether.

As used herein, the term "prophylactic", "prophylaxis", "prevent", "prevention" and the like refers to action taken to prevent a subject from contracting a disease, when a subject is not suspected of having already contracted the disease, but there exists a heightened danger or expectation of contracting the particular disease or disorder in the present or future. The terms further refer to action taken to prevent a subject from contracting any disease, when a subject has already received a vaccination/immunization against a specific disease, the effect of which, however, is not long-lasting. As such, as used herein, an activity would be properly referred to as "prophylactic" or "preventative" as long as the subject in question does not have any disease symptoms but is expected to possibly contract a particular disease or disorder. An action properly initially referred to as "prophylactic" may become "therapeutic" if it turns out following at least one initial administration of a virosome that, despite an initial lack of suspicion that a particular disease or disorder exists in a subject, this subject actually has contracted a particular disease or disorder. Conversely, an activity property initially referred to as "therapeutic" may, if continued beyond curing a particular disease or disorder, become "prophylactic" or "preventative" in nature.

As used herein, the term "pharmaceutical" refers to characteristics of compositions and/or medicaments which render them suitable for administration to a living animal, preferably a human.

As used herein, the terms "potentiating", "immunopotentiating", "stimulating", "immunostimulating", "immunostimulatory" and the like are used interchangeably in the context of an (empty) virosome to refer to a virosome or effect thereof on immune functions which is non-specific in the sense defined above.

As used herein the term "virosome" refers to a reconstituted viral envelope which can be derived from a variety of viruses but which lacks the infectious nucleocapsids and the genetic material of the source virus. A virosome is a special type of lipid vesicle comprising, in its lipid membrane, at least one viral envelope protein. Due to its composition, such structures belong to the class of compounds termed "proteoliposomes", in which the proteins in the lipid membrane are of viral origin. That is, virosomes consist of a mixture of membrane lipids either of viral or non-viral origin and one or more viral envelope proteins.

As used herein, the terms "disease" and "disorder" refer to an abnormality of the body or mind that causes discomfort, dysfunction, or distress and is classified into infectious, non-infectious, neoplastic, immune or metabolic disorder or disease.

As used herein, the terms "naked" and "empty" are used interchangeably with reference to virosomes. The terms refer to the fact that the so-characterized virosomes contain no disease-specific antigen, nor do they bear any in or on their lipid bilayer, other than the at least one influenza viral envelope protein. As such, a "naked" or "empty" lipid vesicle, such as a "naked" or "empty" virosome, means that the only protein or polypeptide comprised in the virosome so designated is the at least one viral envelope protein as defined above. It is noted in this context that the known procedures for producing virosomes as described herein, rarely allow complete removal of all contaminants. A virosome may therefore comprise residual traces of substances involved in its preparation (e.g. trace detergents) and would still be properly understood as "naked" or "empty" in the above sense as long as such trace substances do not elicit any immune response which is "specific" in the above sense.

As used herein, the term "adjuvant" (used as either a noun or an adjective) denotes a secondary substance which is administered in combination (and not necessarily simultaneously) with a primary, active substance responsible for an intended prophylactic and/or therapeutic effect. An "adjuvant" substance therefore does not itself manifest a prophylactic and/or therapeutic effect, but rather supports, promotes or otherwise potentiates the prophylactic and/or therapeutic effect manifested by the primary, active substance.

The designation of a substance or an effect as "adjuvant" depends on the specific context in which this substance is used, or on the specific context in which this effect is manifested, rather than on the intrinsic nature of the substance or effect per se. Therefore, a substance which may in one situation function as an "adjuvant" in the above sense may in another situation function as an active agent, depending on the prophylactic and/or therapeutic effect intended or manifested. By way of illustration, while a virosome which comprises at least one influenza envelope protein in its lipid membrane has been described as an adjuvant in the art, this virosome would not properly be termed an adjuvant in the context of the present invention, since the non-specific immunostimulatory activity is attributed to this virosome. Here, the virosome is the active agent, although another secondary substance supporting, promoting or otherwise potentiating the non-specific prophylactic and/or therapeutic effect of the virosome may be properly termed an adjuvant.

The preparation of virosomes is well-known by the person skilled in the art. For example, suitable protocols for the preparation of virosomes are described, for example, in EP 538437 or, alternatively, in Mischler and Metcalfe (2002). Vaccine 20, B17-23.

For example, virosomes may be reconstituted from original viral membrane lipids and spike glycoproteins after solubilization of, for example, intact influenza virus with octaethyleneglycol monododecyl ether, sedimentation of the nucleocapsid (the viral glycoproteins and lipids will remain in the supernatant), and removal of the detergent in the supernatant with a hydrophobic resin (Bio-Beads SM2) (WO 92/19267).

Preparation of virosomes containing HAs from different strains of parental viruses may be performed with various amounts, including equal amounts of proteins of those parental viruses. Advantageously, parental virus envelope proteins such as HA may be solubilized with the non-ionic detergent octaethyleneglycol monododecyl ether. After removal of the detergent with Bio-Beads SM2, virosomes containing different types of envelope proteins may be formed.

One form of virosome is an immunopotentiating reconstituted influenza virosome ("IRIV" or "influenza virosome"). These are spherical, unilamellar vesicles with a mean diameter on the order of 150 nm, prepared from a mixture of phospholipids and influenza virus surface glycoproteins, but they do not contain any viral nucleic acids. The hemagglutinin ("HA") membrane glycoprotein of influenza virus plays a key role in the mode of action of influenza virosomes. This major antigen of influenza virus is a fusion-inducing component, which facilitates antigen delivery to immunocompetent cells.

Influenza virus subtypes from which the at least one viral envelope protein may advantageously be derived are influenza H1N1, influenza H1N2, influenza H2N2, influenza H3N2, influenza H3N8, influenza H5N1, influenza H5N2, influenza H5N3, influenza H5N8, influenza H5N9, influenza H7N1, influenza H7N2, influenza H7N3, influenza H7N4, influenza H7N7, influenza H9N2 and/or influenza H10N7. Further, the at least one viral envelope protein may advantageously be derived from influenza A/Bangkok/1/79, influenza A/Beijing/32/92, influenza A/Brazil/11/78, influenza A/California/7/2004 (H3N2), influenza A/Chile/1/83, influenza A/Christchurch/4/85, influenza A/England/42/72, influenza A/Fujian/411/2002 (H3N2), influenza A/Guizhou/54/89, influenza A/Hong Kong/1/68, influenza A/Johnannesburg/33/94, influenza A/Leningrad/360/86, influenza A/Mississippi/1/85, influenza A/Moscow/10/99 (H3N2), influenza A/New Caledonia/20/99 (H1N1), influenza A/Panama/2007/99 - RESVIR-17), influenza A/Philippines/2/82, influenza A/Port Chalmers/1/73, influenza A/Scotland/840/74, influenza A/Shangdong/9/93, influenza A/Shanghai/11/87, influenza A/Sichuan/2/87, influenza A/Singapore/6/86, influenza A/Sydney/5/97, influenza A/Texas/1/77, influenza A/USSR/90/77, influenza A/Victoria/3/75, influenza A/Wisconsin/67/2005 (H3N2), influenza A/Wuhan/359/95, influenza A/Wyoming/3/2003 X-147), influenza B/Hong Kong/330/2001, influenza B/Jilin/20/2003, influenza B/Malaysia/2506/2004, influenza B/Shanghai/361/2002, influenza A/Beijing/262/95, influenza B/Victoria/98926/70, influenza B/Singapore/222/79, influenza B/USSR/100/83, influenza B/Yamagata/16/88, influenza B/Panama/45/90, influenza B/Hong Kong/5/72, influenza B/Ann Arbor/1/86, influenza A/Bayern/7/95, influenza B/Shangdong/7/97), and/or B/Jiangsu/10/2003.

Influenza virosomes comprise a spherical lipid membrane, consisting essentially of phospholipid(s), preferably from phosphatidylcholine(s) (PC) and/or phosphatidylethanolamine(s) (PE). In contrast to liposomes, influenza virosomes contain the functional viral envelope glycoproteins HA and/or neuraminidase ("NA") intercalated in the phospholipid bilayer membrane. The biologically active HA not only confers structural stability and homogeneity to virosomal formulations but also significantly contributes to the immunological properties by maintaining the fusion activity of a virus. While it cannot be excluded that HA and/or NA may be recognized as a foreign antigen by the host immune system, any such recognition would only be able to trigger an immunostimulatory response specific for the parental influenza virus. In contrast and as set out above, the present inventors have surprisingly determined that, while providing specific protection against the parental virus, empty virosomes also simultaneously trigger a non-specific response capable of potentiating the host immune system against diseases or disorders which are not associated with or caused by the parental virus or, in the case that the viral envelope proteins are derived from more than one parental virus, parental viruses.

Influenza virosomes act as efficient and highly effective means of non-specifically enhancing the immune response. They are also known to have an excellent safety profile (Schaad et al. (2000). Antimicrob Agents Chemother 44, 1163-1167; Glück et al. (2000). J. Infcet. Dis. 181, 1129-1132), meaning that they are well suitable for use in medications intended for unspecific immunostimulation in humans.

Influenza virosomes can be reconstituted from the original viral membrane lipids and spike glycoproteins after solubilization of inactivated influenza virus with, for example, octaethyleneglycol monododecyl ether, sedimentation of the nucleocapsid (the viral glycoproteins and lipids will remain in the supernatant), and removal of the detergent in the supernatant with a hydrophobic resin (Bio-Beads SM2). Protocols for the preparation of influenza virosomes are given in WO 92/19267 and for generic virosomes in WO 04/071492.

The at least one viral envelope protein is derived from an influenza virus. The virosome used in the present invention may also be a chimeric virosome, meaning that it contains viral envelope proteins, such as hemagglutinin, from at least two different influenza virus strains, for example from influenza strains X-31 and A/Sing or any of the influenza virus strains mentioned above.

The virosome used in the present invention preferably comprises lipids selected from the group consisting of cationic lipids, synthetic lipids, glycolipids, phospholipids cholesterol, or derivatives thereof. Phospholipids comprise preferably phosphatidylcholine, sphingomyelin, phosphatidylethanolamine, phosphatidylserine, phosphatidylglycerol, phosphatidic acid, cardiolipin, and phosphatidylinositol with varying fatty acyl compositions. Cationic lipids are preferably selected from the group consisting of DOTMA (N-[(1-(2,3-dioleyloxy)propyl]-N,N,N-trimethylammonium chloride), DOTAP (N-[1-(2,3-dioleoyloxy)propyl]-N,N,N-trimethylammonium chloride, DODAC (N,N-dioleyl-N,N,-dimethylammonium chloride), DDAB (didodecyldimethylammonium bromide), TC-Chol (cholesteryl N-(trimethylammonioethyl)carbamate chloride), DC-Chol (cholesteryl N-(dimethylammonioethyl)carbamate chloride), or other cationic cholesterol derivatives, and stearylamine or other aliphatic amines and the like. They may be formulated as small unilamellar liposomes in a mixture with PC (phosphafidylcholine). The virosomes used in the present invention may preferably comprise egg-derived PC and, more preferably, 1-oleyl-3-palmitoyl-rac-glycero-2-phosphatidylethanolamine.

Preferably, the virosome used in the invention comprises membrane lipids such as phosphatidylcholine, phoshatidylethanolamine, phosphatidylserine, and/or cholesterol derivatives. In the most preferred embodiment, the virosome further comprises a cationic lipid, for example cationic lipids are preferably selected from the group consisting of DOTMA (N-[(1-(2,3-dioleyloxy)propyl]-N,N,N-trimethylammonium chloride), DOTAP (N-[1-(2,3-dioleoyloxy)propyl]-N,N,N-trimethylammonium chloride, DODAC (N,N-dioleyl-N, N,-dimethylammonium chloride), DDAB (didodecyldimethylammonium bromide), TC-Chol (cholesteryl N-(trimethylammonioethyl)carbamate chloride), DC-Chol (cholesteryl N-(dimethylammonioethyl)carbamate chloride), or other cationic cholesterol derivatives, and stearylamine or other aliphatic amines, DPPE (dipalmitoylphosphatidylethanolamines), DOGS (Dioleoyl-Glycero-Succinate), DOSPA (2,3-dioleoyloxy-N-[2(sperminecarboxamido)ethyl]-N,N-dimethyl-1-propanaminium trifluoroacetate), DOSPER (1,3-dioleoyloxy-2-(6-carboxyspermyl)propylamide), THDOB (N,N,N',N'-tetramethyl-N,N'-bis (2-hydroxyethyl)-2, 3,-dioleoyloxy-1,4- butanediammonium iodide), DOPA (Dioleoyl-sn-Glycero-Phosphate), DOTP (dioctyl tere-phthalate), DOSC (dioleoyl-succinyl-glycerol), DOTB (dioleoyl-e-(4'-trimethylammonio)-butanoyl-sn-glycerol), DOPC (Dioleoyl-sn-Glycero-Phosphocholine) and the like. Especially preferred, the cationic lipid is chosen from cationic cholesterol derivatives such as TC-Chol (cholesteryl N-(trimethylammonioethyl) carbamate) or DC-Chol (cholesteryl N-(dimethylammonioethyl) carbamate).

The membrane of the virosome used in the invention preferably comprises between 1.9 and 37 mol% DC-Chol or TC-Chol, relating to a total lipid content of the membrane. In an especially preferred embodiment, the content of DC-Chol or TC-Chol in the membrane is between 1.9 and 16 mol% of the total lipid content of the membrane. The residual lipid content of the membrane consists preferably of phospholipids, most preferably phosphatidylcholine and phosphytidylethanolamine in a ratio of 4:1. Additionally, the membrane may contain an amount of HA sufficient to guarantee fusion activity of the virosome.

A co-emulsifying agent may also be used in order to improve the rigidity and/or the sealing of the virosome. Examples of co-emulsifying agents are cholesterol esters charged or neutral as cholesterol sulphate, derivatives with a sterol backbone, such as derivatives from vegetable origin, for example sitosterol, sigmasterol, and mixtures thereof.

A virosome used in the invention may for example be obtained by a process analogous to any one of the processes for making DOTAP-containing virosomes disclosed in Examples 1 to 3 and 6 of WO 97/41834, except that DOTAP is replaced by DOSPER and that the DOSPER concentration in the final virosome membrane is properly adjusted as disclosed in WO 97/41834 and, in particular, does not exceed 70% by weight of the total lipid content of the virosome. Basically, a method of preparation of the present virosomes may comprise the following steps:
a) preparing a buffer solution that comprises a non-ionic detergent and that further comprises DOSPER and other lipids and at least one viral envelope protein;
b) adjusting the lipid concentrations to - based on total membrane lipids - 5 to 30% by weight of DOSPER and to a balance of 95 to 70% by weight of said other lipids comprising phosphatidylcholine (PC) or a derivative thereof and optionally phosphatidylethanolamine (PE) and/or cationic lipids other than DOSPER; and
(c) removing the detergent by dialysis or by treating the solution with microcarrier beads, resulting in the formation of said virosomes.

The (at least one) disease or disorder may be an infectious, a non-infectious, a neoplastic, an immune or a metabolic disease or disorder. In one embodiment, the inventive use entails the application of the lipid vesicle used in the invention to healthy subjects facing a temporary increased exposure to one or more infectious diseases or disorders, or of (still) healthy subjects immediately following suspected exposure to one or more infectious diseases or disorders but before appearance of symptoms or confirmation of diagnosis. The classification of an action vis a vis a subject as therapeutic or prophylactic is discussed hereinabove.

The inventive use may also be applied to the treatment of one or more already-existing diseases or disorders, optionally as an independent complementation of specific treatments of such diseases or disorders. Noteworthy in this context is the non-specificity of the immunopotentiation achieved with the virosomes described herein. Here, the non-specificity implies that multiple diseases or disorders may be combated, eliminated and/or prevented simultaneously. Further, the number of diseases and/or disorders which are simultaneously addressable is in principle unlimited. This is a clear advantage over existing vaccination schemes, in which the number of diseases and/or disorders prevented, treated and/or eliminated is limited to the number and type of specific antigens present in the particular vaccine or vaccine cocktail prepared.

According to one embodiment, the at least one infectious disease or disorder may be a viral disease or disorder, a bacterial disease or disorder, a fungal disease or disorder, a parasitic disease, or disorder or a prionic disease or disorder.

According to another embodiment, the viral infectious disease or disorder may advantageously be chosen from AIDS, AIDS Related Complex, Chickenpox (Varicella), Common cold, Cytomegalovirus Infection, Colorado tick fever, Dengue fever, Ebola haemorrhagic fever, Epidemic parotitis, Genital warts, Hand foot and mouth disease, Hepatitis, Herpes simplex, Herpes zoster, HPV. Lassa fever, Measles, Marburg haemorrhagic fever, Infectious mononucleosis, Mumps, Poliomyelitis, Progressive multifocal leukencephalopathy, Rabies, Rubella, SARS, Smallpox (Variola), Viral encephalitis, Viral gastroenteritis, Viral meningitis, Viral pneumonia, West Nile disease, Yellow fever. These and other viral infectious diseases or disorders are caused by RSV, Polioviruses, Rubella virus, Dengue virus, Flaviviridae, Coronaviridae, Reoviridae, Rabies virus, Paramyxoviridae (e.g., mumps virus, measles virus, respiratory syncytial virus, etc.), orthomyxoviridae, simian immunodeficiency virus (SIV), HAV, HBV, HCV, HDV, HEV, HPV, HSV, HIV, CMV, EBV, Polio virus, varicella, Bunyavirus (e.g. La Crosse virus).

According to a further embodiment, the bacterial infectious disease or disorder may advantageously be chosen from Anthrax, Bacterial Meningitis, Brucellosis, Campylobacteriosis, Cat Scratch Disease, Cholera, Diphtheria, Epidemic Typhus, Gonorrhea, Impetigo- Legionellosis, Leprosy (Hansen's Disease), Leptospirosis, Listeriosis, Lyme Disease, Melioidosis, MRSA infection, Nocardiosis, Pertussis (Whooping Cough), Plague, Pneumococcal pneumonia, Psittacosis, Q fever, Rocky Mountain Spotted Fever (RMSF), Salmonellosis, Scarlet Fever, Shigellosis, Syphilis, Tetanus, Trachoma, Tuberculosis, Tularemia, Typhoid Fever, Typhus; Urinary Tract Infections. These and other bacterial infectious diseases or disorders are caused by Corynebacterium diphtheriae, Clostridium tetani, Bordetella pertussis, Neisseria meningitidis, including serotypes Meningococcus A, B, C, Y and W135, Haemophilus influenza type B (Hib), and Helicobacter pylori.

According to another embodiment, the fungal infectious disease or disorder may advantageously be chosen from Aspergillosis, Blastomycosis, Candidiasis, Coccidioidomycosis, Cryptococcosis, Histoplasmosis, Tinea pedis.

According to another embodiment, the parasitic infectious disease or disorder may advantageously be chosen from African trypanosomiasis, Amebiasis, Ascariasis, Babesiosis, Chagas Disease, Clonorchiasis, Cryptosporidiosis, Cysticercosis, Diphyllobothriasis, Dracunculiasis, Echinococcosis, Enterobiasis, Fascioliasis, Fasciolopsiasis, Filariasis, Free-living amebic infection, Giardiasis, Gnathostomiasis, Hymenolepiasis, Isosporiasis, Kala-azar, Leishmaniasis, Malaria, Metagonimiasis, Myiasis, Onchocerciasis, Pediculosis, Pinworm Infection, Scabies, Schistosomiasis, Taeniasis, Toxocariasis, Toxoplasmosis, Trichinellosis, Trichinosis, Trichuriasis, Trypanosomiasis.

According to another embodiment, the prionic infectious disease or disorder may advantageously be chosen from transmissible spongiform encephalopathy, Bovine spongiform encephalopathy, Creutzfeldt-Jakob disease, Kuru.

According to another embodiment, the neoplastic disease or disorder may be a cancer. The cancer may for example be a sarcoma, a leukemia, a lymphoma, a myeloma, a melanoma, an adenoma, a carcinoma, a choriocarcinoma, a gastrinoma, a pheochromocytoma, a prolactinoma, or a neuroma.

Specifically, the cancer may be advantageously selected from Adult Acute Lymphoblastic Leukemia; Childhood Acute Lymphoblastic Leukemia; Acute Myeloid Leukemia; Adrenocortical Carcinoma; Childhood Adrenocortical Carcinoma; AIDS-Related Cancers; AIDS-Related Lymphoma; Anal Cancer; Basal Cell Carcinoma; Extrahepatic Bile Duct Cancer; Bladder Cancer; Bone Cancer, Osteosarcoma/Malignant Fibrous Histiocytoma; Brain Stem Glioma; Adult Brain Tumor; Brain Cancer (e.g. Brain Stem Glioma; Cerebellar Astrocytoma; Cerebral Astrocytoma/Malignant Glioma; Ependymoma; Medulloblastoma; Supratentorial Primitive Neuroectodermal Tumors; Visual Pathway and Hypothalamic Glioma); Breast Cancer (female and male); Bronchial Adenomas/Carcinoids; Burkitt's Lymphoma; Carcinoid Tumor, Gastrointestinal Carcinoid Tumor; Carcinoma of Unknown Primary Origin; Primary Central Nervous System Lymphoma; Cervical Cancer; Childhood Cancers; Chronic Lymphocytic Leukemia; Chronic Myelogenous Leukemia; Chronic Myeloproliferative Disorders; Colon Cancer; Colorectal Cancer; Cutaneous T-Cell Lymphoma, Mycosis Fungoides and Sézary Syndrome; Endometrial Cancer; Ependymoma; Esophageal Cancer; Ewing's Family of Tumors; Extracranial Germ Cell Tumor; Extragonadal Germ Cell Tumor; Extrahepatic Bile Duct Cancer; Eye Cancers (e.g. Intraocular Melanoma Eye Cancer and Retinoblastoma); Gallbladder Cancer; Gastric (Stomach) Cancer;; Gastrointestinal Carcinoid Tumor; Gastrointestinal Stromal Tumor (GIST); Extracranial Germ Cell Tumor; Extragonadal Germ Cell Tumor; Ovarian Germ Cell Tumor; Gestational Trophoblastic Tumor; Gliomas; (e.g. Brain Stem Glioma, Cerebral Astrocytoma, Visual Pathway and Hypothalamic Glioma); Hairy Cell Leukemia; Head and Neck Cancer; Primary Hepatocellular (Liver) Cancer; Hodgkin's Lymphoma; Hypopharyngeal Cancer; Intraocular Melanoma; Pancreatic cancer (Islet Cell Carcinoma); Kaposi's Sarcoma; Kidney (Renal Cell) Cancer; Laryngeal Cancer; Acute Leukemias (e.g. Lymphoblastic Leukemia, Acute Myeloid Leukemia, Chronic Lymphocytic Leukemia, Chronic Myelogenous Leukemia, Hairy Cell Leukemia); Lip and Oral Cavity Cancer; Liver Cancer (primary and metastatic); Non-Small Cell Lung Cancer; Small Cell Lung Cancer; AIDS-Related Lymphoma; Burkitt's Lymphoma; Cutaneous T-Cell Lymphoma; Hodgkin's Lymphoma; Non-Hodgkin's Lymphoma; Primary Central Nervous System Lymphoma; Macroglobulinemia, Waldenström's; Malignant Fibrous Histiocytoma of Bone/Osteosarcoma; Medulloblastoma; Melanoma; Intraocular (Eye) Melanoma; Merkel Cell Carcinoma; Mesothelioma; Primary Metastatic Squamous Neck Cancer with Occult; Multiple Endocrine Neoplasia Syndrome; Multiple Myeloma/Plasma Cell Neoplasm; Mycosis Fungoides; Myelodysplastic Syndromes;, Myelodysplastic/Myeloproliferative Diseases; Chronic Myelogenous Leukemia; Myeloid Leukemia; Multiple Myeloma; Chronic Myeloproliferative Disorders; Nasal Cavity and Paranasal Sinus Cancer; Nasopharyngeal Cancer; Nasopharyngeal Cancer; Neuroblastoma; Oropharyngeal Cancer; Osteosarcoma/Malignant Fibrous Histiocytoma of Bone; Ovarian Cancer; Ovarian Epithelial Cancer; Ovarian Germ Cell Tumor; Ovarian Low Malignant Potential Tumor; Pancreatic Cancer; Islet Cell Pancreatic Cancer; Paranasal Sinus and Nasal Cavity Cancer; Parathyroid Cancer; Penile Cancer; Pheochromocytoma; Pineoblastoma and Supratentorial Primitive Neuroectodermal Tumors, Childhood; Pituitary Tumor; Plasma Cell Neoplasm/Multiple Myeloma; Pleuropulmonary Blastoma; Pregnancy and Breast Cancer; Pregnancy and Hodgkin's Lymphoma; Primary Central Nervous System Lymphoma; Prostate Cancer; Rectal Cancer; Transitional Cell Cancer of Renal Pelvis and Ureter; Retinoblastoma; Rhabdomyosarcoma; Salivary Gland Cancer; Ewing's Family of Tumors; Soft Tissue Sarcoma; Uterine Sarcoma; Sézary Syndrome; Skin Cancer (non-Melanoma); Skin Cancer (Melanoma); Skin Carcinoma, Merkel Cell; Small Cell Lung Cancer; Small Intestine Cancer; Soft Tissue Sarcoma; Metastatic Squamous Cell Carcinoma; Squamous Neck Cancer with Primary Occult; Stomach (Gastric) Cancer; Supratentorial Primitive Neuroectodermal Tumors; Cutaneous T-Cell Lymphoma; Testicular Cancer; Thymoma; Thymoma and Thymic Carcinoma; Thyroid Cancer; Gestational Trophoblastic Tumor,; Carcinoma of Unknown Primary Site; Cancer of Unknown Primary Site; Unusual Childhood Cancers; Urethral Cancer; Endometrial Uterine Cancer; Uterine Sarcoma; Vaginal Cancer; Vulvar Cancer; Waldenström's Macroglobulinemia; Wilms' Tumor; and Women's Cancers.

According to a further embodiment the animal is a mammal. The mammal is preferably a human, a chimpanzee, a cynomologous monkey, a gibbon, a simian monkey, a macaque monkey, a mouse, a rat, a cat, a dog, a horse, a rabbit, a camel, a Ilama, a ruminant, a horse or a pig. A preferred ruminant may be a cow, a bull, a goat, a sheep, a bison, a buffalo, a deer or a stag.

In a further embodiment, the medicament is suitable for administration intramuscularly, intradermally, intraveneously (e.g. by injection), subcutaneously, intraperitoneally, parenterally, topically, endotracheally, intraauracularly, intraarticularly, intraocularly, locally, by a patch (for example as a skin patch), by spray (for example as a nasopharyngeal spray) orally (e.g. as tablets, capsules, caplets, dragees), by suppository (e.g. as rectal suppository or vaginal suppository), by drops (e.g. as eye drops) or mucosally (e.g. intranasally, sublingually, by gargling or by sucking on a lozenge). Accordingly, the medicament may be formulated or confectioned as a solution for injection, a patch, as a spray, as a suppository, as a gargling solution, as a lozenge or as drops. Administration may be in a single dose or, as need dictates, in multiple doses with intervening time intervals as deemed appropriate by the supervising clinician. Intradermal, intramuscular, subcutaneous and intraveneous and mucosal administration is preferred, with intradermal administration being especially preferred.

According to another embodiment, the virosome described herein above is prepared in a form which allows repeated administration to the patient. The combination of the virosomes of the invention with other compounds e.g. adjuvants or immunostimulants may synergistically enhance the overall effect. The amount and type of virosome, the site of stimulation, and co-stimulating signals (infections, exposure to allergens, etc.) define the overall effect. The effect is transient, on the order of hours to weeks. The duration of the effect achieved depends on dose magnitude, dose timing, the route of administration chosen as well as the composition of the medicament administered.

In one embodiment, the virosome as described herein above is prepared in a form which allows it to be administered multiple times before and/or after exposition of an individual to a pathogen. Ideally, preparation of the virosome in a form allowing multiple administrations within an administration window of 3-7 days pre- and/or post-exposition is desired, with a form allowing multiple administrations within an administration window of 5 days pre- and/or post-exposition being preferred. Especially preferred is a form of the virosome allowing multiple administration within an administration window both 5 days pre-exposition as well as 5 days post-exposition, in other words 10 days in total. However, this window may in practice be difficult to gauge, since the exact timepoint of exposition is rarely known with certainty. In such cases, the administration window may be calculated relative to a suspected or expected exposition.

In a further embodiment, formulation of the virosome is such as to allow administration once a day within the pre- and/or post-exposition administration window(s). However, the virosome may be formulated so as to allow more frequent or infrequent administrations within the administration window(s) as needed. For example, the virosome may be administered once, twice, three times, four times or five times a day within the pre- and/or post-exposition administration window(s). Likewise, combinations of these administration frequencies on different days within the pre- and/or post-exposition administration window(s) are also possible.

A medicament containing the virosome described herein above is formulated in pharmaceutically acceptable preparations. Such preparations may routinely contain pharmaceutically acceptable concentrations of salt, buffering agents, preservatives, compatible carriers, supplementary immune potentiating agents such as adjuvants and cytokines and optionally other therapeutic agents. The preferred amount of virosome to be administered depends on the disease or disorder to be prevented, treated and/or eliminated. Generally, doses ranging from about 1 ng/kg to about 100 mg/kg are believed to be effective, said kilograms referring to body weight of the animal treated. The preferred range is believed to be from about 10 ng/kg to about 10 µg/kg. The absolute amount will depend upon a variety of factors, including the composition selected for administration, whether the administration is in single or multiple doses (see above), and individual patient parameters including age, physical condition, size, weight, and the stage of the disease.

The route and regimen of administration will vary depending upon the stage or severity of the disease or disorder to be prevented, treated and/or eliminated, and is to be determined by the skilled practitioner. A medicament containing the virosome described herein above will generally be suitable for parenteral administration. Here, the medicament comprises virosomes dissolved or suspended in an acceptable carrier, preferably an aqueous carrier. A variety of aqueous carriers may be used, e.g. water, buffered water, 0.4% saline, 0.3% glycine, hyaluronic acid and the like. These compositions may be sterilized by conventional, well known sterilization techniques, or may be sterile-filtered. The resulting aqueous solutions may be packaged for use as they are, or lyophilized, the lyophilized preparation being combined with a sterile solution prior to administration.

The medicament containing the virosome as described herein above may additionally contain pharmaceutically acceptable auxiliary substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, among many others. Actual methods for preparing parenterally administrable compounds will be known or apparent to those skilled in the art and are described in more detail in for example, Remington: The Science and Practice of Pharmacy ("Remington's Pharmaceutical Sciences") Gennaro AR ed. 20th edition, 2000: Williams & Wilkins PA, USA.

The medicament containing the virosome as described herein above may be formulated so as to allow administration in oral dosage forms for example as tablets, capsules (each including timed release and sustained release formulations), pills, powders, granules, elixirs, tinctures, solutions, suspensions, syrups and emulsions, or by injection. For instance, for oral administration in the form of a tablet or capsule, the active drug component can be combined with an oral, non-toxic pharmaceutically acceptable inert carrier such as ethanol, glycerol, water and the like.

Similarly, the medicament containing the virosome as described herein above may be formulated so as to allow administration intravenously (either by bolus or infusion methods), intraperitoneally, subcutaneously, topically with or without occlusion, or intramuscularly. In preferred embodiments, the medicament prepared according to the inventive use is administered intramuscularly, subcutaneously, intradermally, mucosal or transdermally. All of these forms are well known to those of ordinary skill in the pharmaceutical arts.

The dosage regimen according to which the medicament containing the virosome as described herein above may be administered is selected in accordance with a variety of factors, including for example species, age, weight, sex and medical condition of the patient, the stage and severity of the disease or disorder to be prevented, treated and/or eliminated, and the particular type of virosome employed. A physician of ordinary skill in the art can readily determine and prescribe the effective amount of the medicament required to prevent, counter, or arrest the progress of a malignancy or infectious disease or disorder. Optimal precision in achieving concentration of drug with the range that yields efficacy either without toxicity or with acceptable toxicity requires a regimen based on the kinetics of the lipid vesicle's availability to target sites. This process involves a consideration of the distribution, equilibrium, and elimination of the virosome, and is within the ability of the skilled practitioner and can be addressed with no more than routine experimentation.

The daily dose of a medicament containing the virosome as described herein above may be varied over a range from 10 ng/kg to about 10 µg/kg of virosomes per adult per day. For oral administration, the medicament prepared according to the inventive use is preferably provided in the form of tablets containing from 0.001 to 1,000 mg, preferably 0.001, 0.01, 0.05, 0.1, 0.5, 1, 2.5, 10, 20, 50, 100 milligrams of virosome for the symptomatic adjustment of dosage according to signs and symptoms of the patient in the course of treatment. An effective amount of virosome in the medicament prepared according to an embodiment the inventive use is ordinarily supplied at a dosage level of from about 0.0001 mg/kg to about 50 mg/kg of body weight per day. More particularly, the range is from about 0.0001 mg/kg to 7 mg/kg of body weight per day.

Furthermore, a medicament containing the virosome as described herein above may be formulated so as to allow administration in intranasal form, or via transdermal routes known to those of ordinary skill in the art. If the formulation allows administration in the form of a transdermal delivery system, the administration dosage will be continuous rather than intermittent throughout the dosage regimen.

The medicament prepared according to the inventive use may be coupled to a class of biodegradable polymers useful in achieving controlled release of a drug, for example, polylactic acid, polyepsilon caprolactone, polyhydroxy butyric acid, polyorthoesters, polyacetals, polydihydro-pyrans, polycyanoacrylates, and cross-linked or amphipathic block copolymers of hydrogels.

A suitable formulation of the medicament prepared according to the inventive use for topical administration may be, for example, in the form of a solution, cream, ointment, gel, lotion, shampoo, or aerosol formulation adapted for application to the skin. These topical pharmaceutical compositions containing the medicament prepared according to the inventive use ordinarily include about 0.02% to 5% by weight of the active compound, i.e. the virosome, in admixture with a pharmaceutically acceptable vehicle.

Generally, the medicament comprising a virosome as described herein above may generally comprise 0.02 wt%, 0.05 wt%, 0.08 wt%, 1 wt%, between 1 wt% and 2 wt%, 2 wt%, between 2 wt% and 3 wt%, 3 wt%, between 3 wt% and 4 wt%, 4 wt%, between 4 wt% and 5 wt% or 5 wt% of the virosome.

Regardless of the route by which the medicament prepared according to the inventive use is administered, it is to be administered in an effective amount. An effective amount is that amount of a pharmaceutical preparation that, alone or together with further doses, stimulates the desired non-specific immunostimulatory response.

Moreover, when desired or necessary, suitable binders, lubricants, disintegrating agents and coloring agents can also be incorporated into the medicament as prepared by the inventive use. Suitable binders include, without limitation, starch, gelatin, natural sugars such as glucose or beta-lactose, corn sweeteners, natural and synthetic gums such as acacia, tragacanth or sodium alginate, carboxymethyl cellulose, polyethylene glycol, waxes and the like. Lubricants used in these dosage forms include, without limitation, sodium oleate, sodium stearate, magnesium stearate, sodium benzoate, sodium acetate, sodium chloride and the like. Disintegrators include, without limitation, starch, methylcellulose, agar, bentonite, xanthan gum and the like.

The liquid forms of the medicament as prepared by the inventive use may be suitably flavored by suspending or dispersing agents such as the synthetic and natural gums, for example, tragacanth, acacia, methyl cellulose and the like. Other dispersing agents, which may be employed, are glycerin and the like. For parenteral administration, sterile suspensions and solutions are desired. Isotonic preparations, which generally contain suitable preservatives, are employed when intravenous administration is desired. Topical preparations containing the active drug component can be admixed with a variety of carrier materials well known in the art, such as, for example, alcohols, aloe vera gel, allatoin, glycerin, vitamins A or E oils, mineral oil, PPG2 myristoyl propionate, and the like, to form, for example, alcoholic solutions, topical cleansers, cleansing creams, skin gels, skin lotions, and shampoos in cream or gel formulations.

In one embodiment, the medicament as prepared by the inventive use may further comprise at least one adjuvant enhancing and/or mediating an immune response, for example an innate immune response, a Th₁ or Th₂ response. Suitable adjuvants may enhance the immunological response by activating macrophages and/or stimulating specific sets of lymphocytes. A suitable adjuvant may be any ligand suitable for the activation of a pathogen recognition receptor (PRR). Immune response-potentiating compounds are classified as either adjuvants (in the sense defined herein above) or cytokines. Adjuvants may enhance the immunological response by providing a reservoir of antigen (extracellularly or within macrophages), activating macrophages and stimulating specific sets of lymphocytes.

Adjuvants of many kinds are well known in the art; specific examples include Freund's (complete and incomplete), mycobacteria such as BCG, M. vaccae, or Corynebacterium parvum, Cholera toxin or tetanus toxin, E.coli heat-labile toxin, quil-saponin mixtures such as QS-21 (SmithKline Beecham), MF59 (Chiron) and various oil/water emulsions (e.g. IDEC-AF). Other adjuvants which may be used include, but are not limited to mineral salts or mineral gels such as aluminum hydroxide, aluminum phosphate, and calcium phosphate; surface active substances such as lysolecithin, pluronic polyols, polyanions, peptides, keyhole limpet hemocyanins, and dinitrophenol, immunostimulatory molecules, such as saponins, muramyl dipeptides and tripeptide derivatives, short nucleic acid stretches such as CpG dinucleotides, CpG oligonucleotides, monophosphoryl Lipid A, and polyphosphazenes, particulate and microparticulate adjuvants, such as emulsions, liposomes, cochleates, or immunostimulating complex adjuvants.

Cytokines are also useful due to their lymphocyte stimulatory properties. Many cytokines useful for such purposes will be known to one of ordinary skill in the art, including interteukin-2 (IL-2), IL-12, GM-CSF and many others. Furthermore ligands from the chemokine family, such as RANTES (Regulated upon Activation Normal T cell Expressed and Secreted), a lipoprotein of Gram-positive bacteria, a yeast cell wall component, a double-stranded RNA, a lipopolysaccharide of Gram-negative bacteria, flagellin, a U-rich single-stranded viral RNA, , a Suppressor 6f Cytokine Signalling small interfering RNA (SOCS siRNA), a Pan DR epitope (PADRE) and mixtures thereof are suitable.

For prevention, treatment and/or elimination of cancers and/or metastases, the medicament containing the virosome as described herein above may be prepared so as to allow administeration in combination with a pharmaceutically acceptable carrier adopted for topical administration. Additionally, for the prevention, treatment and/or elimination of cancer, tumors and/or metastases, or viral infections, the medicament as prepared by the inventive use may be used together with other agents known to be useful in treating such malignancies. For combination treatment with more than one active agent, where the active agents can be administered concurrently, the active agents can be administered concurrently, or they can be administered separately at staggered times.

It is to be understood that elements of the various embodiments set out herein above are freely combinable with one another within any particular aspect of the invention, as well as between one or more aspects of the invention. For instance, though elements of a particular embodiment may be formally discussed in the context of the use of a virosome for the preparation of a medicament, it will be readily understood that elements of this embodiment are freely combinable with elements from other embodiments discussed within the context of this use.

### Brief description of the figures

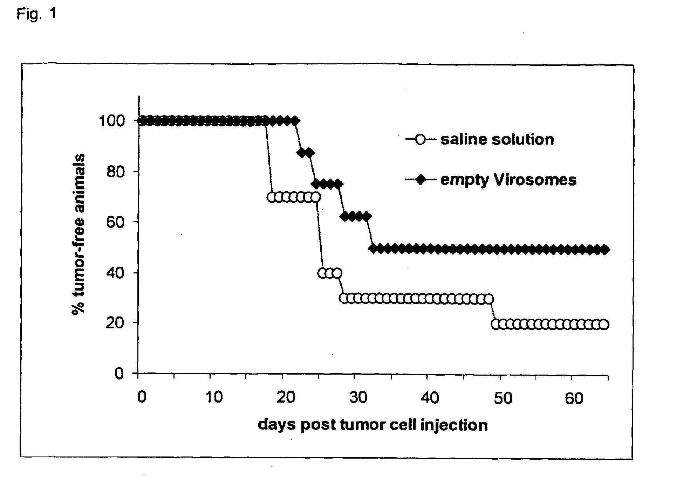

- Fig. 1: Effect of naked virosomes on tumor growth in mice. The figure shows the results of monitoring tumor growth in mice which had been injected with tumor cells and, subsequently, weekly with one of two samples: saline solution as a control (open circles); and empty virosomes (solid diamonds). The results show that injection with naked, i.e. empty virosomes resulted in significantly more tumor-free mice at the end of the experimental period than injection with the control.

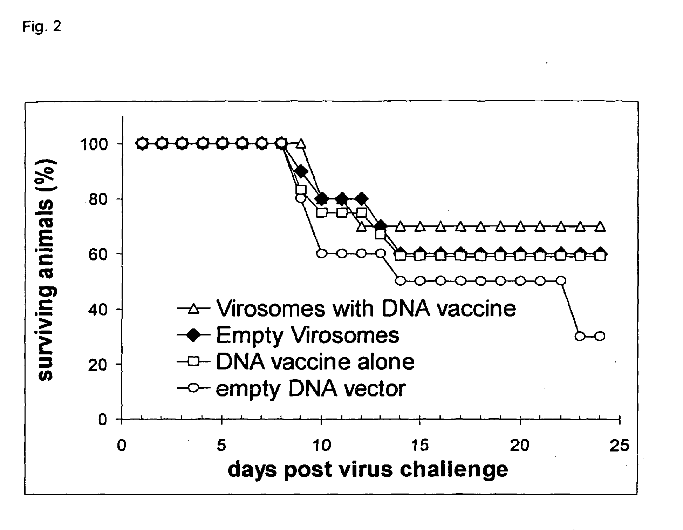

- Fig. 2: Protective effect of naked virosomes on progression of La Crosse virus. The figure shows the results of monitoring the survival of mice challenged with La Crosse virus. The mice had been injected with one of four samples prior to viral challenge: virosomes encapsulating DNA encoding La Crosse virus glycoproteins (open triangles); empty virosomes (solid diamonds); DNA vector alone, said vector comprising DNA expressing La Crosse virus glycoproteins (open squares); DNA vector alone, said vector lacking DNA expressing La Crosse virus glycoproteins (open circles). The results indicate that treatment with naked virosomes resulted in an improved survival rate as compared to the DNA vector control lacking DNA expressing La Crosse virus glycoproteins.

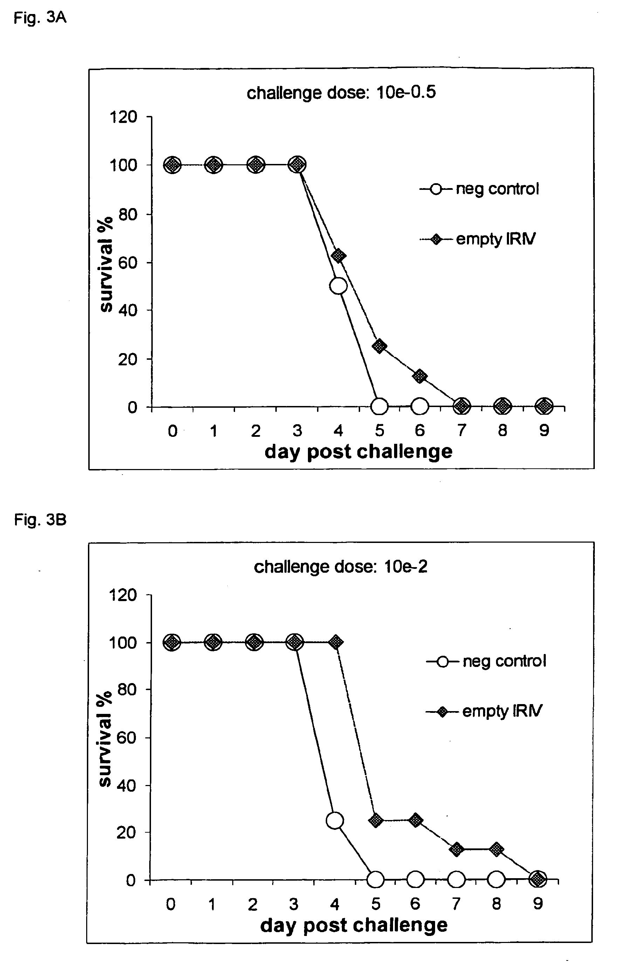

- Figs. 3A, 3B: Protective effect of empty virosomes on progression of Leptospira interrogans infection. The figure shows the result of monitoring the survival rate of gerbils challenged with the bacterium Leptospira interrogans. The gerbils had been injected with empty influenza virosomes (shaded diamonds) prior to bacterial challenge. As a negative control "untreated" gerbils were challenged (open circles).

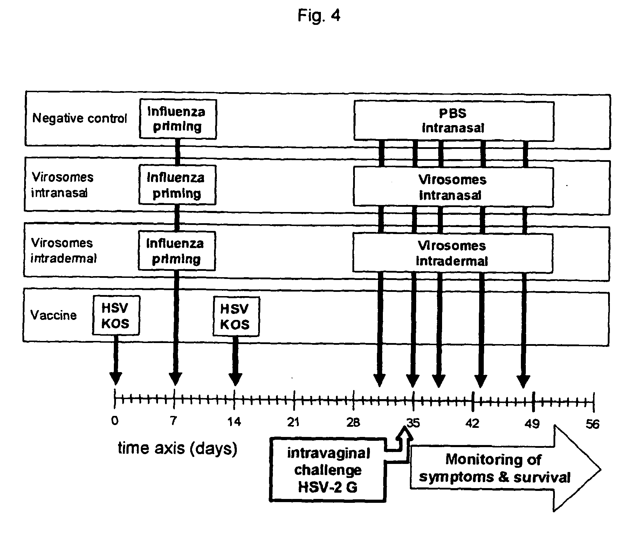

- Fig.4: Experimental design overview of HSV-2 challenge experiment. The figure shows that empty virosomes were administered both intradermally and intranasally multiple times both prior to as well as following lethal challenge with HSV-2.

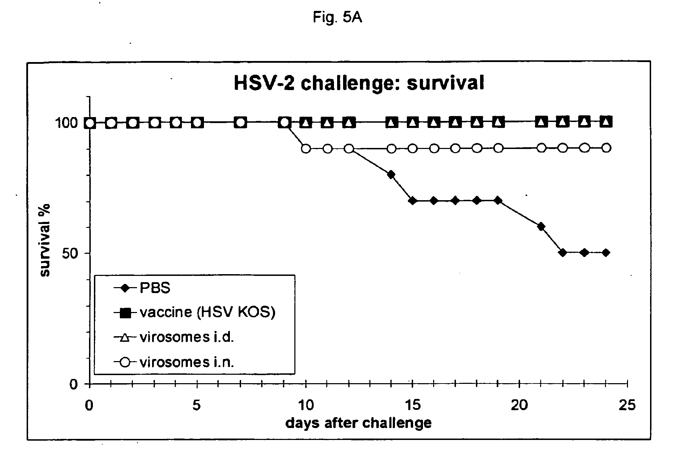

- Fig.5A: Effect of empty virosomes on overall survival in the progression of HSV-2 infection as compared to PBS control- and HSV vaccine-treated mice. Groups of 10 mice were challenged at day 0 with HSV-2 G strain administered into the vagina. Empty virosomes were administered both intradermally ("i.d.", open triangles) and intranasally ("i.n.", open circles). The survival rate of mice receiving PBS control is shown in solid diamonds, and that of mice having received HSV vaccine 34 and 20 days prior to challenge is shown in solid squares. The results indicate that treatment with empty virosomes results in an improved survival rate as compared to PBS-treated control-treated mice. Surprisingly, the survival rate observed in mice treated intradermally with empty virosomes is equal to that observed in mice which received prior HSV-specific vaccination. Further, mice which received empty virosomes intranasally exhibited a 25-day survival rate which was only 10% worse than that observed in mice which received prior HSV-specific vaccination.

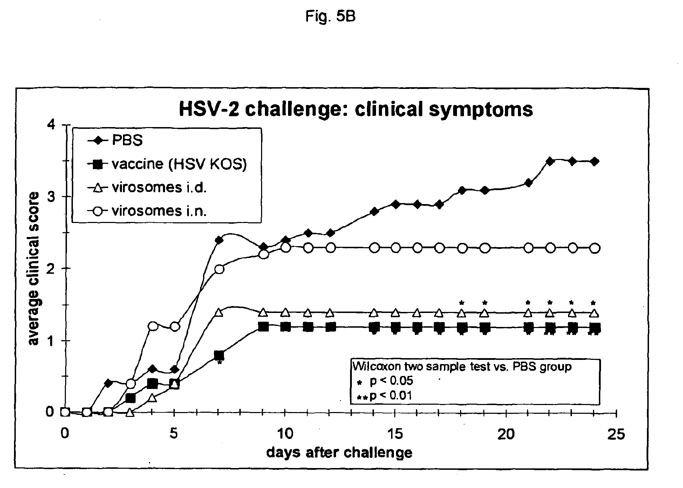

- Fig. 5B: Effect of empty virosomes on clinical score in the progression of HSV-2 infection as compared to PBS control- and HSV vaccine-treated mice. Groups of 10 mice were challenged at day 0 with HSV-2 G strain administered into the vagina. Empty virosomes were administered both intradermally ("i.d.", open triangles) and intranasally ("i.n.", open circles). The clinical score of mice receiving PBS control is shown in solid diamonds, and that of mice having received HSV-2 vaccine 34 and 20 days prior to challenge is shown in solid squares. The results indicated that mice treated with empty virosomes experienced less severe disease symptoms than mice treated with PBS control following exposition (challenge) to HSV- 2 virus. Notably, the severity of disease symptoms in mice having received empty virosomes by an intradermal route of administration was only slightly worse than in those which had previously received HSV-specific vaccine.
- Fig. 6: Overview diagram showing certain characteristics of and the interrelationship between the innate and adaptive arms of the immune system.

The specific embodiments of the invention and the following examples are provided to demonstrate the efficiency of the claimed invention but are not to be interpreted as limiting the scope of the invention. To the extent that specific materials are mentioned, it is merely for purposes of illustration and is not intended to limit the invention. Unless otherwise specified, biochemical and molecular biology procedures, such as those set forth in Voet, Biochemistry, Wiley, 1990; Stryer, Biochemistry, W.H. Freeman, 1995; Bodanszky, Peptide Chemistry. A Practical Textbook, 2nd ed., Springer-Vertag, Bertin. 1993; Sambrook et al., Molecular Cloning, Cold Spring Harbor Laboratory, 2001; Ausubel et al. (Eds.), Current Protocols in Molecular Biology, John Wiley & Sons, 2000 are used.

### Example 1 (preparation example): Reagents used in preparation and working examples

Reagents: Octaethyleneglycol-mono-(n-decyl)ether (OEG, C₁₂E₈), is purchased from Fluka Chemie GmbH-(Buchs, Switzerland). Sucrose (Eur. Phar.) is purchased from Merck (Dietikon, Switzerland). Egg phosphatidyl choline (PC) is obtained from Lipoid (Cham, Switzerland). 1-Oleoyl-3-palmitoyl-rac-glycero-2-phosphoethanolamine is obtained from Bachem (Bubendorf, Switzerland). Bio-Beads SM2 are purchased from Bio-Rad Laboratories (Glattbrugg, Switzerland). Cholesterol N-(trimethylammonioethyl)carbamate chloride (TC-chol) was purchased from Merck Eprova (Schaffhausen, Switzerland).

Influenza viruses of the A/Singapore/6/86 (A/Sing) strain and other influenza A.strains, propagated in the allantoic cavity of embryonated eggs (Gerhard, W. (1976), J. Exp. Med. 144:985-995), were obtained from Bema Biotech AG (Bem, Switzerland) and purified as described (Skehel, J. et al.,(1971). Virology 44:396). The hemagglutinin/phospholipid ratio was determined according to Böttcher (Böttcheret al. (1961). Anal. Chim. Acta 24, 203) and HA-quantification after SDS-PAGE with the Coomassie-extraction method as described by Ball (Ball (1986). Anal. Biochem. 155, 23).

### Example 2 (preparation example): Preparation of the standard virosomes as influenza virosomes

For a final volume of 4 ml, 32 mg egg PC and 8 mg OPPE are dissolved in 3 ml of PBS, 100 mM OEG (PBS/OEG). 2 mg HA of inactivated influenza A/Singapore/6/86 virus or another influenza A strain is centrifuged at 100,000 x g for 1 h at 4°C and the pellet is dissolved in 1 ml of PBS/OEG. The detergent solubilized phospholipids and viruses are mixed and sonicated for 1 min. This mixture is centrifuged at 100,000 x g for 1 h at 18°C. Virosomes are formed by detergent removal using two times 1.5 g of wet SM2 Bio-Beads for 1 h each at room temperature with shaking. The freshly formed virosomes are then sterile filtered (0.22 µm).

### Example 3 (preparation example): Preparation of virosomes containing TC-Chol

Virosomes containing TC-Chol are prepared by the detergent removal method. For a final volume of 4 ml, 32 mg egg PC, 8 mg OPPE and 5 mg cholesteryl N-(trimethylammonioethyl)carbamate chloride (TC-chol) are dissolved in 2.6 ml of PBS, 100 mM OEG (PBS/OEG). 2 mg HA of inactivated A/Singapore/6/86 influenza virus or another influenza A strain is centrifuged at 100,000 x g for 1 h at 4°C and the pellet is dissolved in 1 ml of PBS/OEG. The detergent solubilised phospholipids and viruses are mixed with 0.4 mL of 50% (w/v) sucrose and sonicated for 1 min. This mixture is centrifuged at 100,000 x g for 1 h at 18°C. Virosomes are formed by detergent removal using two times 1.5 g of wet SM2 Bio-Beads for 1 h each at room temperature with shaking. The freshly formed virosomes are then sterile filtered (0.22 µm) and aliquoted in sterile glas vials. The closed vials are frozen at -70°C and then lyophilized at -40°C for 20 h and 10°C for 2 h. The closed vials are stored at frozen until use.

### Example 4 (working example): Non-specific immunostimulation with emptyvirosomes decreases tumor burden in mice

The following experiment was performed in the context of the development of a cancer vaccine for therapeutic use. The vaccine is intended to be applied while the tumor is already developing in the body. It is intended that immunization with virosomes enhances the specific immune response against the tumor to a level sufficient for complete elimination of the growing tumor cells.

Eight to ten week-old female C5781/6 mice were pre-immunized with inactivated influenza virus containing 1 µg hemagglutinin to simulate the situation in pre-immunized humans who have become naturally infected with influenza at some previous time in life. Three weeks later, 10,000 B16 tumor cells were injected subcutaneously. Two days later, immunization with approx. 1-25 mg empty virosomes per kg body weight were started and continued at weekly intervals for 9 weeks.

The experiment included two treatment groups. Mice in each respective group were treated as follows:
Group 1: Phosphate-buffered saline ("saline solution" in Fig.1; open circles)
Group 2: Standard (empty) virosomes composed of egg-derived phospholipids and solubilized influenza envelopes ("empty virosomes" in Fig.1; solid diamonds)

During the treatment period, the animals were closely monitored for the development of tumors. Mice that had developed palpable tumors were euthanized. Accordingly, the readout of the study was the number of tumor-free mice per group throughout the observation period. The results are shown in Fig. 1. As can be taken from Fig.1, 50% of the mice treated with empty virosomes were significantly protected (solid diamonds in Fig.1; "empty virosomes"), while only 20% of the mice treated with saline control remained tumor-free (open circles in Fig. 1; "saline solution").

In conclusion, administration of naked virosomes led to the eradication of tumors in a significant number of tumor-burdened mice.

### Example 5 (working example): Non-specific immunostimulation with naked virosomes increases survival rate in mice infected with La Crosse virus

The experiment was originally performed to assess virosomes as a delivery vehicle for a DNA-based vaccine against La Crosse virus. Plasmid DNA encoding viral glycoproteins of LaCrosse virus (VR1012-G1 G2) is packaged into virosomes in order to increase plasmid uptake and expression of viral antigens. One of the administered controls was naked virosomes.

Genetically modified mice lacking the receptor for type I interferons (IFNAR-I -/- mice) are more susceptible to viral infections and are thus a suitable challenge model for La Crosse virus. The mice were pre-immunized against influenza to simulate the situation in pre-immunized humans, who have become naturally infected with influenza at some previous time in life, and a single dose of the prototype La Crosse vaccine (or control formulations as indicated below) was administered ten days later. The challenge with live virus at a dosage of 200,000 infectious units-was performed 4 weeks later. The survival of the animals was monitored for 24 days after infection. The following groups were included in the experiment:
1. DNA vector expressing La Crosse glycoproteins combined with Virosomes ("Virosomes with DNA vaccine" in Fig. 2; open triangles)
2. Virosomes without DNA ("Empty Virosomes" in Fig. 2; solid diamonds)
3. DNA vector alone, the vector expressing La Crosse virus glycoproteins ("DNA vaccine alone" in Fig. 2; open squares)
4. DNA vector alone, the vector not expressing La Crosse virus glycoproteins ("empty DNA vector" in Fig. 2; open circles)

The results of the study are shown in Fig. 2. As can be taken from Fig. 2, empty virosomes provided equally good protection as the DNA vector expressing La Crosse virus glycoproteins when administered alone. In each case, 60% of the animals were protected. In comparison, the group receiving DNA vector plus virosomes showed a protection rate of 70%. As a negative control, the DNA vector NOT expressing La Crosse virus glycoproteins was administered. This still resulted in a protection rate of 30%.

The three week interval between immunization and challenge may be necessary for the development of adaptive immunity, but it is likely suboptimal to assess a non-specific protective effect manifested by naked virosomes. Despite this suboptimal administration, however, while not as effective as virosomes encapsulating La Crosse-derived DNA, initial immunization with naked (i.e. empty) virosomes still elicited a non-specific immunostumulatory effect sufficient to improve survival rate by about 30% as compared with the control.

### Example 6 (working example): Non-specific immunostimulation with naked virosomes increases survival rate in gerbils infected with the bacterium Leptospira interrogans

The experiments were performed in the context of the development of a prophylactic vaccine against Leptospira interrogans. Two different challenge doses of Leptospira interrogans were used. Virosomes were prepared as outlined in example 2 ("empty influenza virosomes").

Gerbils were immunized two weeks prior to challenge with live bacteria. The challenge was performed with different dosages of the pathogen, as indicated in the separate Figs. 3A and 3B.
1. negative control: untreated animals ("neg control" in Figs. 3A and 3B; open circles)
2. empty virosomes (empty influenza virosomes" in Figs. 3A and 3B; shaded diamonds)

The results are shown in Figs. 3A and 3B. As can be taken from Figs. 3A and 3B, while none of the animals survived the bacertial infection, animals which had been pre-treated with empty virosomes survived longer than the untreated control animals.

### Example 7 (working example): Non-specific immunostimulation with naked virosomes increases survival rate and lessens severity of disease in mice infected with herpes simplex virus 2 ("HSV-2")

The experiment was performed to assess and confirm the unspecific protective effect of influenza virosomes against infectious diseases that are completely unrelated to the antigenic components of virosomes. In the present experiment, HSV-2 was used as the challenge pathogen. The experimental protocols were based on previously established challenge models in mice for *Herpes simplex virus type* 2 (HSV-2). The mice used in all experiments were female Balb/C mice aged 6-8 weeks at the start of the experiments.

The virosomes used in the present experiment were prepared as described in Example 2, above. The readout of the experiments consisted of daily clinical scores and survival. The protective effect of virosomes was determined by comparison of the groups receiving virosomes to groups receiving control treatments of phosphate buffered saline ("PBS"). In addition, the HSV-2 challenge included a positive control group, which received a conventional live-attenuated vaccine designed to induce a specific (adaptive) immunity with a known protective effect.

In the setting of a specific vaccine it has been demonstrated repeatedly that the adjuvant effect of virosomes is substantially enhanced by pre-existing immunity. Thus, it was decided to use pre-immunized animals in all the experiments. To that end, the animals were immunized 4 weeks before challenge with an intramuscular injection of purified, inactivated Influenza virus containing 1 microgram HA resuspended in PBS. As explained above in Example 4, the mice in the present experiment were also pre-immunized with inactivated influenza virus containing 1 µg hemagglutinin to simulate the situation in pre-immunized humans who have become naturally infected with influenza at some previous time in life.

The timing of virosome application was based on the assumption that the non-specific protective effect afforded by virosomes is most likely short lived. Repeated administrations shortly before and after challenge with HSV-2 thus appeared the most reasonable approach to detect the non-specific effect. At the same time, the selected time interval between virosome administration and challenge with HSV-2 (5 days or less) was chosen to be clearly too short for fully boosting the specific immunity against influenza (7-14 days), thereby ruling out the possibility in subsequent analysis that any effect observed could be attributable to a specific immunization against influenza due to the virosomal HA proteins. The virosome doses applied ranged from 20 to 40 micrograms HA and represented the near-maximal dose technically possible for the chosen routes of application.

The live-attenuated vaccine for the vaccine-control mice was administered according to previously established schedules and dosages. The mice were immunized intradermally into their right footpad twice with 8,000 plaque forming units ("pfu") of the attenuated HSV KOS strain at day 0 of the study and boosted with 400,000 pfu at day 14. These animals were not pre-immunized against influenza in order to avoid possible interference with the specific vacccination. One week before challenge all groups received a subcuntaneous injection of 5 mg Medroxyprogesterone acetate (Depo Provera 150, Pfizer) to block their menstrual cycle.

At day 34, all mice were challenged with a lethal virus dose (1000 pfu) of HSV-2 G strain administered into the vagina in a volume of 10 µl, and the clinical symptoms and survival were subsequently recorded daily.

An overview of the treatment with virosomes or PBS control is shown in Fig. 4. As shown in Fig.4, the mice received a total of five doses of empty virosomes or PBS control both before and after HSV-2 challenge itself. Specifically empty virosomes or PBS control were administered once prior to HSV-2 challenge, whereas the remaining four administrations were performed after challenge. The intranasal application was performed under sedation with Xylazine (0.3 mg/animal), and 10 µl were applied into each nostril, resulting in a total dose of 20 µg HA for each intranasal application. Each intradermal application comprised an injection of 40 µl containing 40 µg HA.

After challenge, the animals were observed daily and a clinical score was assigned to each animal in accordance with a published scoring scheme (Natuk et al. (2006). Journal of Virology 80(9), 4447-57). The clinical scoring scheme is shown below as Table 1, with higher clinical score rankings indicating more severe disease symptoms, and lower clinical score rankings indicating less severe disease symptoms.

**Table 1: Clinical scoring scheme according to Natuk et al. (2006)**

| **symptoms** | **clinical score** |
|---|---|
| no signs of disease | 0 |
| vaginal erythema a | 1 |
| vaginal erythema and edema | 2 |
| vaginal herpetic lesions | 3 |
| genital ulceration, hair loss, unilateral paralysis | 4 |
| bilateral paralysis or death | 5 |

Moribund animals showing paralysis were euthanized. The animals were observed for 25 days after challenge. The raw data were subsequently analyzed for differences between groups with regard to survival and daily clinical scores. Statistical analysis was performed using the Wilcoxon rank sum test as for example published in "Statistische Prinzipien for medizinische Projekte" ("Statistical principles for medical projects"), by J. Hüsler & H. Zimmermann; 4th Edition, 2006; Verlag Hans Huber, Hogrefe AG, Bern; pages 116-121.

The results relating to the survival of the following groups of mice from days 0-25 after challenge are shown in Fig. 5A:
1. Mice receiving PBS control ("PBS" in Fig. 5A; solid diamonds);
2. Mice receiving live-attenuated HSV-specific vaccine ("vaccine (HSV KOS)" in Fig. 5A; solid squares);
3. Mice receiving empty virosomes by an intradermal route of administration ("virosomes i.d." in Fig. 5A; open triangles); and
4. Mice receiving empty virosomes by an intranasal route of administration ("virosomes i.n." in Fig. 5A; open circles).

Fig. 5A clearly shows that the 25-day survival of PBS-treated control mice following HSV challenge was only 50%, while that of the HSV vaccine-treated mice was 100%. Surprisingly, administration of naked virosomes via an intradermal route resulted in a survival rate of 100% against HSV infection at day 25, which was equivalent to the protection afforded by HSV-specific preimmunization. The protection afforded by empty virosomes administered via an intranasal route was only slightly worse, at 90%. Overall, then, it can be concluded that empty/naked virosomes are able to provide protection against diseases which are not associated with the viral envelope protein(s) comprised in/on the virosomes; i.e. enoty/naked virosomes are able to elicit an immunopotentialtion which is non-specific. Further, the magnitude of the immunopotentiation elicited is of the same magnitude as afforded by specific pre-immunization.

The results relating to the clinical score of the same groups of mice as described above in the context of Fig. 5A are shown in Fig. 5B. Fig. 5B clearly shows that the 25-day clinical score of PBS-treated control mice following HSV challenge was about 3.5 (more severe), while that of the HSV vaccine-treated mice was slightly over 1 (less severe). Importantly, intradermally administered empty virosomes reduced the severity of disease symptoms relative to PBS-treated control mice almost down to levels corresponding to mice which had been previously treated with HSV-specific vaccine. In contrast, mice which received empty virosomes via an intranasal route of administration experienced disease symptoms of a severity only slightly less than those experienced by PBS control -treated mice. It therefore appears that an intradermal administration of empty virosomes has a more potent immunopotentiating effect than intranasal administration of empty virosomes.

Overall, in can be concluded that both intradermal as well as intranasal administration of empty virosomes increased the survival rate to HSV infection in mice as well as lessened the severity of this infection in mice relative to mice also infected with HSV but having received a PBS control instead of naked virosomes. This non-specific protective effect was greater by both measures for intradermally than for intranasally administered empty virosomes, with the protective effect achieved using intradermally administered virosomes being equivalent or only slightly less than that achieved by a specific HSV vaccine. This protection is non-specific, since the empty virosomes administered bear viral envelope proteins (HA) from influenza, but no HSV-specific antigens. Finally, it should be noted that the challenge dose used in the above experiment corresponds to 1 LD50 ("lethal dose 50%", meaning the dose at which 50% of the animals are expected to die). A higher challenge dose or a more virulent challenge strain may yield more distinctive results. Further, the dosage and the schedule of application for empty virosomes were speculative. For this reason, the observed protective effects may not reflect the full protective potential of empty virosomes.

## Claims

1. Use of a virosome comprising, in its lipid membrane, at least one viral envelope protein, for the preparation of a medicament for non-specifically stimulating the immune response of an animal to prevent and/or effect therapy of a neoplastic, viral or bacterial disease or disorder,
wherein said neoplastic, viral or bacterial disease or disorder is not associated with or caused by the virus from which the at least one viral envelope protein is derived,
wherein the virosome is an empty virosome which comprises no drug or substance associated with said disease or disorder, and
wherein the at least one viral envelope protein is an influenza virus envelope protein.

2. The use according to claim 1, wherein the at least one influenza viral envelope protein is hemagglutinin (HA) or neuraminidase (NA).

3. The use according to claim 1, wherein the influenza viral envelope proteins are hemagglutinin (HA) and neuraminidase (NA).

4. The use according to any of claims 1-3, wherein the neoplastic disease or disorder is a cancer.

5. The use according to claim 4, wherein the cancer is a sarcoma, a leukemia, a lymphoma, a myeloma, a melanoma, an adenoma, a carcinoma, a choriocarcinoma, a gastrinoma, a pheochromocytoma, a prolactinoma, adult T-cell leukemia/lymphoma or a neuroma.

6. The use according to any of claims 1-5, wherein the medicament is for non-specifically stimulating the immune response to at least two diseases or disorders simultaneously.

7. The use according to any of claims 1-6, wherein the animal is a mammal.

8. The use according to claim 7, wherein the mammal is selected from a human, a chimpanzee, a cynomologous monkey, a gibbon, a simian monkey, a macaque monkey, a mouse, a rat, a cat, a dog, a horse, a rabbit, a camel, a llama, a ruminant, a horse, a pig.

9. The use according to claim 8, wherein the ruminant is chosen from a cow, a bull, a goat, a sheep, a bison, a buffalo, a deer and a stag.

10. The use according to any of claims 1-9, wherein the medicament is:
• suitable for administration intraveneously, intramuscularly, intradermally, subcutaneously, intraperitoneally, parenterally, topically, locally, orally, sublingually or by gargling and/or
• formulated or confectioned as a solution for injection, as a patch, as a spray, as a suppository, as a gargling solution or as drops.

11. The use according to claim 1-10, wherein the virosome is an immunopotentiating reconstituted influenza virosome ("influenza virosome"), or a chimeric virosome.

12. The use according to claim 11, wherein the influenza virosome or the chimeric virosome is a chimeric influenza virosome.

13. A virosome comprising, in its lipid membrane, at least one viral envelope protein, for use in non-specifically stimulating the immune response of an animal to prevent and/or effect therapy of a neoplastic, viral or bacterial disease or disorder,
wherein said neoplastic, viral or bacterial disease or disorder is not associated with or caused by the virus from which the at least one viral envelope protein is derived,
wherein the virosome is an empty virosome which comprises no drug or substance associated with said disease or disorder, and
wherein the at least one viral envelope protein is an influenza virus envelope protein.

14. The virosome according to claim 13, wherein the at least one influenza viral envelope protein is hemagglutinin (HA) or neuraminidase (NA).

15. The virosome according to claim 13, wherein the influenza viral envelope proteins are hemagglutinin (HA) and neuraminidase (NA).

16. The virosome according to any of claims 13-15, wherein the neoplastic disease or disorder is a cancer.

17. The virosome according to claim 16, wherein the cancer is a sarcoma, a leukemia, a lymphoma, a myeloma, a melanoma, an adenoma, a carcinoma, a choriocarcinoma, a gastrinoma, a pheochromocytoma, a prolactinoma, adult T-cell leukemia/lymphoma or a neuroma.

18. The virosome according to any of claims 13-17, wherein the lipid vesicle is for non-specifically stimulating the immune response to at least two diseases or disorders simultaneously.

19. The virosome according to any of claims 13-18, wherein the animal is a mammal.

20. The virosome according to claim 19, wherein the mammal is selected from a human, a chimpanzee, a cynomologous monkey, a gibbon, a simian monkey, a macaque monkey, a mouse, a rat, a cat, a dog, a horse, a rabbit, a camel, a llama, a ruminant, a horse, a pig.

21. The virosome according to claim 20, wherein the ruminant is chosen from a cow, a bull, a goat, a sheep, a bison, a buffalo, a deer and a stag.

22. The virosome according to any of claims 13-21, wherein the medicament is:
• suitable for administration intraveneously, intramuscularly, intradermally, subcutaneously, intraperitoneally, parenterally, topically, locally, orally, sublingually or by gargling and/or
• formulated or confectioned as a solution for injection, as a patch, as a spray, as a suppository, as a gargling solution or as drops.

23. The virosome according to claim 22, wherein the virosome is an immunopotentiating reconstituted influenza virosome ("influenza virosome"), or a chimeric virosome.

24. The virosome according to claim 23, wherein the influenza virosome or the chimeric virosome is a chimeric influenza virosome.

## Patentansprüche

1. Verwendung eines Virosoms, das in seiner Lipidmembran mindest ein virales Hüllprotein umfasst, zur Herstellung eines Medikaments zum unspezifischen Stimulieren der Immunantwort eines Tieres, um eine neoplastische, virale oder bakterielle Erkrankung oder Störung zu verhindern und/oder deren Behandlung zu bewirken,
wobei die neoplastische, virale oder bakterielle Erkrankung oder Störung mit dem Virus, von dem das mindestens eine virale Hüllprotein abgeleitet ist, nicht in Zusammenhang steht oder durch dieses verursacht ist,
wobei es sich bei dem Virosom um ein leeres Virosom handelt, das weder ein Arzneimittel noch eine Substanz, das bzw. die mit der Erkrankung oder Störung in Zusammenhang steht, umfasst, und
wobei es sich bei dem mindestens einen viralen Hüllprotein um ein Hüllprotein von Influenzavirus handelt.

2. Verwendung gemäß Anspruch 1, wobei es sich bei dem mindestens einen Hüllprotein von Influenzavirus um Hämagglutinin (HA) oder Neuraminidase (NA) handelt.

3. Verwendung gemäß Anspruch 1, wobei es sich bei den Hüllproteinen von Influenzavirus um Hämagglutinin (HA) und Neuraminidase (NA) handelt.

4. Verwendung gemäß einem der Ansprüche 1 - 3, wobei es sich bei der neoplastischen Erkrankung oder Störung um eine Krebserkrankung handelt.

5. Verwendung gemäß Anspruch 4, wobei es sich bei der Krebserkrankung um ein Sarkom, eine Leukämie, ein Lymphom, ein Myelom, ein Melanom, ein Adenom, ein Karzinom, ein Chorionkarzinom, ein Gastrinom, ein Phäochromozytom, ein Prolaktinom, adulte T-Zell-Leukämie/Lymphom oder ein Neurom handelt.

6. Verwendung gemäß einem der Ansprüche 1 - 5, wobei das Medikament zum unspezifischen Stimulieren der Immunantwort auf mindestens zwei Erkrankungen oder Störungen gleichzeitig dient.

7. Verwendung gemäß einem der Ansprüche 1 - 6, wobei es sich bei dem Tier um ein Säugetier handelt.

8. Verwendung gemäß Anspruch 7, wobei das Säugetier aus einem Menschen, einem Schimpansen, einem Javaneraffen ("cynomologous monkey"), einem Gibbon, einem Eigentlichen Affen ("simian monkey"), einem Makaken ("macaque monkey"), einer Maus, einer Ratte, einer Katze, einem Hund, einem Pferd, einem Kaninchen, einem Kamel, einem Lama, einem Wiederkäuer, einem Pferd, einem Schwein ausgewählt ist.

9. Verwendung gemäß Anspruch 8, wobei der Wiederkäuer aus einer Kuh, einem Stier, einer Ziege, einem Schaf, einem Bison, einem Büffel, einem Reh und einem Hirsch gewählt ist.

10. Verwendung gemäß einem der Ansprüche 1 - 9, wobei das Medikament
• zur intravenösen, intramuskulären, intradermalen, subkutanen, intraperitonealen, parenteralen, topischen, lokalen, oralen, sublingualen Verabreichung oder Verabreichung durch Gurgeln geeignet und/oder
• als Injektionslösung, als Pflaster, als Spray, als Suppositorium, als Gurgellösung oder als Tropfen formuliert oder konfektioniert ist.

11. Verwendung gemäß Anspruch 1-10, wobei es sich bei dem Virosom um ein immunverstärkendes, rekonstituiertes Influenza-Virosom ("Influenza-Virosom") oder ein chimäres Virosom handelt.

12. Verwendung gemäß Anspruch 11, wobei es sich bei dem Influenza-Virosom oder dem chimären Virosom um ein chimäres Influenza-Virosom handelt.

13. Virosom, das in seiner Lipidmembran mindest ein virales Hüllprotein umfasst, zur Verwendung beim unspezifischen Stimulieren der Immunantwort eines Tieres, um eine neoplastische, virale oder bakterielle Erkrankung oder Störung zu verhindern und/oder deren Behandlung zu bewirken,
wobei die neoplastische, virale oder bakterielle Erkrankung oder Störung mit dem Virus, von dem das mindestens eine virale Hüllprotein abgeleitet ist, nicht zusammenhängt oder durch dieses verursacht ist,
wobei es sich bei dem Virosom um ein leeres Virosom handelt, das weder ein Arzneimittel noch eine Substanz, das bzw. die mit der Erkrankung oder Störung in Zusammenhang steht, umfasst, und
wobei es sich bei dem mindestens einen viralen Hüllprotein um ein Hüllprotein von Influenzavirus handelt.

14. Virosom gemäß Anspruch 13, wobei es sich bei dem mindestens einen Hüllprotein von Influenzavirus um Hämagglutinin (HA) oder Neuraminidase (NA) handelt.

15. Virosom gemäß Anspruch 13, wobei es sich bei den Hüllproteinen von Influenzavirus um Hämagglutinin (HA) und Neuraminidase (NA) handelt.

16. Virosom gemäß einem der Ansprüche 13-15, wobei es sich bei der neuroplastischen Erkrankung oder Störung um eine Krebserkrankung handelt.

17. Virosom gemäß Anspruch 16, wobei es sich bei der Krebserkrankung um ein Sarkom, eine Leukämie, ein Lymphom, ein Myelom, ein Melanom, ein Adenom, ein Karzinom, ein Chorionkarzinom, ein Gastrinom, ein Phäochromozytom, ein Prolaktinom, adulte T-Zell-Leukämie/Lymphom oder ein Neurom handelt.

18. Virosom gemäß einem der Ansprüche 13-17, wobei das Lipidvesikel zum unspezifischen Stimulieren der Immunantwort auf mindestens zwei Erkrankungen oder Störungen gleichzeitig dient.

19. Virosom gemäß einem der Ansprüche 13-18, wobei es sich bei dem Tier um ein Säugetier handelt.

20. Virosom gemäß Anspruch 19, wobei das Säugetier aus einem Menschen, einem Schimpansen, einem Javaneraffen ("cynomologous monkey"), einem Gibbon, einem Eigentlichen Affen ("simian monkey"), einem Makaken ("macaque monkey"), einer Maus, einer Ratte, einer Katze, einem Hund, einem Pferd, einem Kaninchen, einem Kamel, einem Lama, einem Wiederkäuer, einem Pferd, einem Schwein ausgewählt ist.

21. Virosom gemäß Anspruch 20, wobei der Wiederkäuer aus einer Kuh, einem Stier, einer Ziege, einem Schaf, einem Bison, einem Büffel, einem Reh und einem Hirsch gewählt ist.

22. Virosom gemäß einem der Ansprüche 13 - 21, wobei das Medikament
• zur intravenösen, intramuskulären, intradermalen, subkutanen, intraperitonealen, parenteralen, topischen, lokalen, oralen, sublingualen Verabreichung oder Verabreichung durch Gurgeln geeignet und/oder
• als Injektionslösung, als Pflaster, als Spray, als Suppositorium, als Gurgellösung oder als Tropfen formuliert oder konfektioniert ist.

23. Virosom gemäß Anspruch 22, wobei es sich bei dem Virosom um ein immunverstärkendes, rekonstituiertes Influenza-Virosom ("Influenza-Virosom") oder ein chimäres Virosom handelt.

24. Virosom gemäß Anspruch 23, wobei es sich bei dem Influenza-Virosom oder dem chimären Virosom um ein chimäres Influenza-Virosom handelt.

## Revendications

1. Utilisation d'un virosome comprenant, dans sa membrane lipidique, au moins une protéine d'enveloppe virale, pour la préparation d'un médicament pour la stimulation non spécifique de la réponse immunitaire d'un animal pour prévenir et/ou effectuer le traitement d'une maladie ou d'un trouble néoplasique, viral ou bactérien,
dans laquelle ladite maladie ou ledit trouble néoplasique, viral ou bactérien n'est pas associé à ou provoqué par le virus dont dérive l'au moins une protéine d'enveloppe virale,
dans laquelle le virosome est un virosome vide qui ne comprend pas de médicament ou de substance associé à ladite maladie ou audit trouble, et
dans laquelle ladite au moins une protéine d'enveloppe virale est une protéine d'enveloppe de virus grippal.

2. Utilisation selon la revendication 1, dans laquelle l'au moins une protéine d'enveloppe de virus grippal est l'hémagglutinine (HA) ou la neuraminidase (NA) .

3. Utilisation selon la revendication 1, dans laquelle les protéines d'enveloppe de virus grippal sont l'hémagglutinine (HA) et la neuraminidase (NA).

4. Utilisation selon l'une quelconque des revendications 1 à 3, dans laquelle la maladie ou le trouble néoplasique est un cancer.

5. Utilisation selon la revendication 4, dans laquelle le cancer est un sarcome, une leucémie, un lymphome, un myélome, un mélanome, un adénome, un carcinome, un choriocarcinome, un gastrinome, un phéochromocytome, un prolactinome, une leucémie/un lymphome à cellules T de l'adulte ou un neurome.

6. Utilisation selon l'une quelconque des revendications 1 à 5, dans laquelle le médicament est destiné à une stimulation non spécifique de la réponse immunitaire à au moins deux maladies ou troubles simultanément.

7. Utilisation selon l'une quelconque des revendications 1 à 6, dans laquelle l'animal est un mammifère.

8. Utilisation selon la revendication 7, dans laquelle le mammifère est choisi parmi un humain, un chimpanzé, un singe cynomolgus, un gibbon, un singe simien, un singe macaque, une souris, un rat, un chat, un chien, un cheval, un lapin, un chameau, un lama, un ruminant, un cheval, un cochon.

9. Utilisation selon la revendication 8, dans laquelle le ruminant est choisi parmi une vache, un taureau, une chèvre, un mouton, un bison, un buffle, un chevreuil et un cerf.

10. Utilisation selon l'une quelconque des revendications 1 à 9, dans laquelle le médicament est :
• adapté à une administration par voie intraveineuse, intramusculaire, intradermique, sous-cutanée, intrapéritonéale, parentérale, topique, locale, orale, sublinguale ou par gargarisme, et/ou
• formulé ou confectionné sous la forme d'une solution injectable, d'un timbre, d'un aérosol, d'un suppositoire, d'une solution pour gargarisme ou de gouttes.

11. Utilisation selon les revendications 1 à 10, dans laquelle le virosome est un virosome grippal reconstitué immuno-potentialisant ("virosome grippal") ou un virosome chimère.

12. Utilisation selon la revendication 11, dans laquelle le virosome grippal ou le virosome chimère est un virosome grippal chimère.

13. Virosome comprenant, dans sa membrane lipidique, au moins une protéine d'enveloppe virale, pour utilisation dans la stimulation non spécifique de la réponse immunitaire d'un animal pour prévenir et/ou effectuer le traitement d'une maladie ou d'un trouble néoplasique, viral ou bactérien,
dans lequel ladite maladie ou ledit trouble néoplasique, viral ou bactérien n'est pas associé à ou provoqué par le virus dont dérive l'au moins une protéine d'enveloppe virale,
lequel virosome est un virosome vide qui ne comprend pas de médicament ou de substance associé à ladite maladie ou audit trouble, et
dans lequel ladite au moins une protéine d'enveloppe virale est une protéine d'enveloppe de virus grippal.

14. Virosome selon la revendication 13, dans lequel l'au moins une protéine d'enveloppe de virus grippal est l'hémagglutinine (HA) ou la neuraminidase (NA).

15. Virosome selon la revendication 13, dans lequel les protéines d'enveloppe de virus grippal sont l'hémagglutinine (HA) et la neuraminidase (NA).

16. Virosome selon l'une quelconque des revendications 13 à 15, dans lequel la maladie ou le trouble néoplasique est un cancer.

17. Virosome selon la revendication 16, dans lequel le cancer est un sarcome, une leucémie, un lymphome, un myélome, un mélanome, un adénome, un carcinome, un choriocarcinome, un gastrinome, un phéochromocytome, un prolactinome, une leucémie/un lymphome à cellules T de l'adulte ou un neurome.

18. Virosome selon l'une quelconque des revendications 13 à 17, dans lequel le vésicule lipidique est destiné à une stimulation non spécifique de la réponse immunitaire à au moins deux maladies ou troubles simultanément.

19. Virosome selon l'une quelconque des revendications 13 à 18, dans lequel l'animal est un mammifère.

20. Virosome selon la revendication 19, dans lequel le mammifère est choisi parmi un humain, un chimpanzé, un singe cynomolgus, un gibbon, un singe simien, un singe macaque, une souris, un rat, un chat, un chien, un cheval, un lapin, un chameau, un lama, un ruminant, un cheval, un cochon.

21. Virosome selon la revendication 20, dans lequel le ruminant est choisi parmi une vache, un taureau, une chèvre, un mouton, un bison, un buffle, un chevreuil et un cerf.

22. Virosome selon l'une quelconque des revendications 13 à 21, dans lequel le médicament est :
• adapté à une administration par voie intraveineuse, intramusculaire, intradermique, sous-cutanée, intrapéritonéale, parentérale, topique, locale, orale, sublinguale ou par gargarisme, et/ou
• formulé ou confectionné sous la forme d'une solution injectable, d'un timbre, d'un aérosol, d'un suppositoire, d'une solution pour gargarisme ou de gouttes.

23. Virosome selon la revendication 22, lequel virosome est un virosome grippal reconstitué immuno-potentialisant ("virosome grippal") ou un virosome chimère.

24. Virosome selon la revendication 23, dans lequel le virosome grippal ou le virosome chimère est un virosome grippal chimère.
